(19) Europäisches Patentamt
European Patent Office
Office européen des brevets

(11) EP 1 435 915 B1

(12) EUROPÄISCHE PATENTSCHRIFT

(45) Veröffentlichungstag und Bekanntmachung des Hinweises auf die Patenterteilung:
**23.07.2008 Patentblatt 2008/30**

(21) Anmeldenummer: **02782857.3**

(22) Anmeldetag: **08.10.2002**

(51) Int Cl.:
***A61K 9/22*** $^{(2006.01)}$ ***A61K 9/52*** $^{(2006.01)}$
***A61K 31/445*** $^{(2006.01)}$ ***A61K 47/12*** $^{(2006.01)}$

(86) Internationale Anmeldenummer:
**PCT/EP2002/011253**

(87) Internationale Veröffentlichungsnummer:
**WO 2003/030869 (17.04.2003 Gazette 2003/16)**

(54) **ORALE DARREICHUNGSFORMEN FÜR PROPIVERIN ODER SEINEN PHARMAZEUTISCH ANNEHMBAREN SALZEN MIT VERLÄNGERTER WIRKSTOFFFREISETZUNG**

ORAL DOSAGE FORMS FOR PROPIVERINE OR ITS PHARMACEUTICALLY ACCEPTABLE SALTS WITH AN EXTENDED RELEASE OF THE ACTIVE INGREDIENT

FORMES D'ADMINISTRATION ORALE DE PROPIVERINE OU DE SES SELS PHARMACEUTIQUEMENT ACCEPTABLES, A LIBERATION PROLONGEE DE L'AGENT ACTIF

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **09.10.2001 DE 10149674**

(43) Veröffentlichungstag der Anmeldung:
**14.07.2004 Patentblatt 2004/29**

(73) Patentinhaber: **APOGEPHA ARZNEIMITTEL GmbH**
**D-01309 Dresden (DE)**

(72) Erfinder:
- **GRAMATTE, Thomas**
  **01326 Dresden (DE)**
- **GRUBER, Peter**
  **79249 Merzhausen (DE)**
- **GÜLDNER, Peter**
  **01309 Dresden (DE)**
- **HESCHEL, Michael**
  **01796 Pirna (DE)**
- **PAMPERIN, Dirk**
  **6522 CP Nijmegen (NL)**
- **PLOEN, Jan**
  **01326 Dresden (DE)**
- **SCHEITHAUER, Steffen**
  **01326 Dresden (DE)**
- **WEHNER, Wolfgang**
  **01259 Dresden (DE)**

(74) Vertreter: **Glas, Holger**
**Maiwald Patentanwalts GmbH**
**Elisenhof**
**Elisenstrasse 3**
**80335 München (DE)**

(56) Entgegenhaltungen:

| | |
|---|---|
| **EP-A- 1 123 705** | **EP-A- 1 273 301** |
| **WO-A-00/44353** | **WO-A-01/34579** |
| **WO-A-01/62236** | **WO-A-02/060415** |
| **WO-A-02/067906** | **DE-A- 10 016 356** |

- **IWATSUBO: "Clinical effects of propiverine hydrochloride for unhibited neurogenic bladder disorders" NISHINIHON JOURNAL OF UROLOGY, Bd. 52, Nr. 2, 1990, Seiten 233-240, japan**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue orale Darreichungsformen von Propiverin oder seinen pharmazeutisch annehmbaren Salzen mit verlängerter Wirkstofffreisetzung.

Propiverin - der chemische Name ist: 2,2-Diphenyl-2-(1 -propoxy)-essigsäure-(1 -methyl-piperid-4-yl)-ester- oder eines seiner pharmazeutisch annehmbaren Salze sind zur Behandlung hypertoner Funktionszustände im Bereich der Harnblase (überaktive Blase) allgemein bekannt (DE 2937589).

Das Blasenspasmolytikum Propiverin wirkt als Anticholinergikum, indem es die cholinerg/muskarinerg innervierte glatte Muskulatur der Harnblase durch Blockade der entsprechenden Rezeptoren ruhigstellt. Zudem besteht ein im Sinne einer Wirkungsverstärkung gerichteter Einfluß auf den Kalziumhaushalt der Zelle.

Propiverin ist in Form seines Hydrochlorids in schnell freisetzenden oralen Darreichungsformen seit Jahren in verschiedenen Präparaten, z. B. Mictonorm® im Handel. Die bisherige Gabe von z. B. dreimal täglich einem Dragee Mictonorm® à 15 mg Propiverinhydrochlorid führt zu relativ stark fluktierenden Blutspiegeln mit mehrfach täglichen Spitzen. Infolge der anticholinergen Wirkung des Propiverins müssen bei raschem Anstieg des Blutspiegels typische anticholinerge Nebenwirkungen, wie Mundtrockenheit und Akkommodationsstörungen in Kauf genommen werden. Diese Nebenwirkungen begrenzen somit bei unmodifizierten, rasch freisetzenden Darreichungsformen die Menge der möglichen Einmalgabe auf 10 - 20 mg.

Für einen therapeutisch wirksamen Blutspiegel von Propiverin ist somit die exakte Einhaltung der Einnahmeintervalle notwendig, was insbesondere bei älteren Patienten, der Hauptgruppe mit hypertonen Funktionszuständen der Harnblase, zu Problemen führt.

Aus diesen genannten Gründen ist es wünschenswert, die mehrmalige Gabe pro Tag mit all ihren bekannten Effekten auf eine einmalige Gabe bei gleicher oder verbesserter therapeutischer Wirkung zu reduzieren.

Um geeignete orale Darreichungsformen mit ausreichend verzögerter Wirkstofffreisetzung und einem therapeutisch wirksamen Blutspiegel über ein 24-Stunden-Intervall realisieren zu können, ist zu beachten, dass solche Formulierungen naturgemäß wesentliche Anteile ihres Wirkstoffgehalts in tieferen Darmabschnitten freigeben.
Aus pharmakokinetischer Sicht ist allgemein bekannt, dass die Resorptionsgeschwindigkeit im wesentlichen den zeitlichen Aufbau eines Blutspiegels bestimmt und dass das quantitative Ausmaß der Resorption sowie die Halbwertszeit der Substanz im Organismus darüber entscheiden, ob am Ende eines Dosierungsintervalls, d. h. vor Gabe der nächsten Dosis, wirksame Blutspiegel aufrecht erhalten werden können. Eine ausreichende Resorptionsgeschwindigkeit und ein ausreichendes Ausmaß der Wirkstoffresorption über den gesamten Bereich des Magen-Darmtraktes mit seinen unterschiedlichen pH-Werten sind somit notwendig.

Das schwach basische Propiverin mit einem pKa-Wert von 7,35 $\pm$ 0,1 (Wasser, 25 °C) sollte nach allgemeiner Lehrmeinung in protonierter Form im Magen relativ schlecht, dagegen als Base, d. h. als schwach basische Neutralform, im Darmtrakt relativ gut resorbiert werden.

Da aber die innere Darmoberfläche mit einer mikroskopisch dünnen Wasserschicht überzogen ist, sind neben den Säure-Basen-Eigenschaften einer Substanz die Löslichkeit und die Lipophilie der zu resorbierenden Base von ausschlaggebender Bedeutung.

Der Verteilungskoeffizient und die Löslichkeit von Propiverinhydrochlorid in Abhängigkeit vom pH-Wert sind bekannt (Bestimmung des Gehaltes an Propiverin in der wäßrigen Phase mittels HPLC).

Verteilungskoeffizient von Propiverinhydrochlorid (1-Octanol/Wasser, 25 °C)

| pH | K [Mittelwerte, Dreifachmessung] | Log [K] |
|---:|---:|---:|
| 1,0 | 22 | 1,3 |
| 5,0 | 13 | 1,1 |
| 6,0 | 227 | 2,4 |
| 6,5 | 884 | 2,9 |
| 6,8 | 6904 | 3,8 |
| 7,0 | 10346 | 4,0 |
| 7,2 | 15438 | 4,2 |
| 7,5 | 26068 | 4,4 |
| 8,0 | 52372 | 4,7 |

Löslichkeit von Propiverinhydrochlorid in Wasser

| pH | Temperatur [°C] | Löslichkeit [g/l] |
|---|---|---|
| 5,8 | 24 | > 200 |
| 5,8 | 37 | 368 |
| 6,0 | 24 | 209 |
| 7,0 | 24 | 1,2 |
| 7,2 | 24 | 1,1 |

Diese physikochemischen Eigenschaften von Propiverinhydrochlorid erweisen sich somit für die Realisierung einer Retardformulierung als ungeeignet, da im wässrigen Milieu nur im sauren Bereich, dem für Propiverin, wegen der vorliegenden Protonierung ungünstigen Resorptionsbereich, eine ausreichende Löslichkeit vorhanden ist. Dagegen besteht in dem für die Resorption günstigeren pH-Bereich oberhalb pH = 6,65 praktisch Unlöslichkeit, und die lipophile Propiverinbase fällt aus. Im weiteren ist aus praktischen Erfahrungen bekannt, dass bei beginnendem Basenausfall bereits kleinste Mengen von Propiverinhydrochlorid mit unlöslicher Propiverinbase überzogen werden, und eine weitere Überführung in die korrespondierende Base unterbleibt. Diese Substanzeigenschaften des Propiverinhydrochlorids lassen die Realisierung eines 12- oder 24-Stunden-Depots wenig aussichtsreich erscheinen. Aus eben diesen erwähnten Gründen scheiterten Versuche zur Realisierung von transdermalen Systemen (Biol. Pharm. Bull. 1995, 18(7), 968 - 975).

Im weiteren ist bekannt, dass Propiverin einen außerordentlich starken first-pass-Effekt durch Monooxygenasen zum unerwünschten, den Organismus belastenden Propiverin-N-Oxid unterliegt. Das Propiverin-N-Oxid mit seinem quartären, permanent positiv geladenen Stickstoff ist im Gegensatz zu Propiverin im gesamten pH-Bereich sehr gut wasserlöslich (Löslichkeit von > 127 bis > 99 g/l bei pH=4,0 - 8,0) und somit schlechter resorbierbar.

Die im Darm vorhandenen Monooxygenasen oxidieren die im Gleichgewicht vorhandene Propiverinbase zum N-Oxid. Damit ist die Ausscheidung von Propiverin über sein wasserlösliches N-Oxid möglich. Auch diese pharmakokinetische Substanzeigenschaft des Propiverins läßt die Realisierung eines in tieferen Darmabschnitten freisetzenden 24-Stunden-Depots mit einem therapeutisch wirksamen Blutspiegel bzw. Bioäquivalenz zur schnell freisetzenden Handelsform wenig erfolgreich erscheinen. Neben Propiverin sind zur Behandlung hypertoner Funktionszustände der Harnblase die tertiären Amine Oxybutynin und Tolterodin allgemeiner Therapiestandard. Die Halbwertszeit, als wesentliches pharmakokinetisches Kriterium beträgt für die schnellfreisetzenden Handelsformen von Oxybutynin und Tolterodin nur ca. 2 - 3 Stunden, für die von Propiverin dagegen ca. 15 Stunden.

Mittels verschiedener galenischer Techniken wurden deshalb orale Darreichungsformen mit verzögerter Wirkstofffreisetzung von Oxybutynin (US 5912268, WO 9523593, WO 9612477, WO 9637202) und Tolterodin (WO 0012069, WO 0027369) realisiert.

Im Falle von Propiverin scheint eine solche Darreichungsform wenig sinnvoll zu sein, bzw. die lange Halbwertszeit kann einer erfolgreichen Realisierung einer therapeutisch brauchbaren Formulierung sogar entgegenstehen.

Für schwach basische Arzneistoffe sind orale Darreichungsformen mit verzögerter Wirkstofffreisetzung als Single-Unit-Formulierung und auch als Multiple-Unit-Formulierung allgemeiner Stand der Technik.

In kontrolliert freisetzenden Single-Unit-Formen, beispielsweise Matrixtabletten, Mehrschichttabletten, Diffussionstabletten, wird eine zusätzliche Diffussionskontrolle, z. B. mittels Weinsäure (Int. J. Pharm. 1997, 157, 181-187) oder mittels Bernsteinsäure (Pharm. Ind. 1991, 53, 686-690) erzielt. Beim Zerkauen einer solchen monolithischen Form würde aber trotz Retardierung eine hohe Wirkstoffmenge plötzlich freigesetzt, was im Falle hochwirksamer Anticholinergika hinsichtlich der Arzneimittelsicherheit problematisch werden könnte.

Multiple-Unit-Darreichungsformen besitzen diese Nachteile nicht und sind für schwach basische Arzneistoffe ebenfalls beschrieben (Pharm. Ind. 1989, 51, 98 - 101, 540 - 543; ebenda 1991, 53, 69 - 73, 595 - 600, 778 - 785; Arzneim.-Forsch./Drug Res. 1998, 48, 540 - 604). Weitere technische Lösungen für pH-abhängige, im basischen Bereich schlecht lösliche Verbindungen existieren z. B. für Dipyridamol (EP 32562; EP 68191) und für Bromhexin (EP 69259).

WO 00 44 353 beschreibt pharmazeutische Zusammensetzungen zur langsamen Freisetzung eines Wirkstoffs, wie z.B. Propiverin, im Magen-Darm-Trakt. Mit dem Wirkstoff und einem magen und darmsaft unlöslichen Polymer wird eine Teilchenmatrix gebildet, die eine geringe Porengrösse aufweist und zusätzlich umhüllt wird.

Aufgabe der Erfindung ist es, trotz nachteilig erscheinender physikochemischer und pharmakokinetischer Eigenschaften von Propiverin und seinen Salzen erstmals orale Darreichungsformen dieses Wirkstoffes mit verlängerter Freisetzung herzustellen, die unabhängig vom pH-Wert des gesamten Magen-Darmtraktes, unabhängig von eventuellen Störungen der Magen- und Darmperistaltik sowie relativ unabhängig von inter- und intraindividuellen Unterschieden der Patienten über einen Zeitraum von mindestens 24 Stunden einen konstanten, zur Behandlung der überaktiven Blase klinisch relevanten Blutspiegel bei gleichzeitig verminderter Nebenwirkungsrate aufweisen.

Die Lösung der erfindungsgemäßen Aufgabe ergibt sich aus den Merkmalen des unabhängigen Anspruchs. Vorteilhafte Ausführungsformen sind in den abhängigen Unteransprüchen definiert. Gemäß einer bevorzugten Ausführungsform wird die Aufgabe dadurch gelöst, dass man orale Darreichungsformen realisiert, die Propiverin und/oder eines seiner pharmazeutisch annehmbaren Salze in therapeutisch wirksamer Menge, entsprechend 4 mg bis 60 mg Propiverin, enthalten, und folgende in vitro-Freisetzungsraten - gemessen in 750 ml 0,1 N HCl während der ersten Stunde und nachfolgend gemessen in 750 ml USP-Puffer pH = 5,8 unter Anwendung der Ph. Eur. Drehkörbchen-Methode bei 100 U/min und 37 °C - aufweisen:

| | | |
|---|---|---|
| 0 - 20 % | Propiverin, freigesetzt nach | 1 Stunde |
| 10 - 45 % | Propiverin, freigesetzt nach | 3 Stunden |
| 30 - 60 % | Propiverin, freigesetzt nach | 5 Stunden |
| 40 - 75 % | Propiverin, freigesetzt nach | 7 Stunden |
| 50 - 80 % | Propiverin, freigesetzt nach | 9 Stunden |
| > 60 % | Propiverin, freigesetzt nach | 12 Stunden, besonders bevorzugt |
| 60 -90 % | Propiverin, freigesetzt nach | 12 Stunden, |

wobei diese einen klinisch relevanten Blutspiegel über einen langen Zeitraum, Bioäquivalenz zum schnellfreisetzenden Handelspräparat, eine verringerte Nebenwirkungsrate und letztlich durch die Möglichkeit der Einmalgabe eine verbesserte Patientenkompliance erwarten lassen.

Erstmals konnten Nachteile, resultierend aus den physikochemischen und pharmakokinetischen Eigenschaften des Wirkstoffes Propiverin und Vorurteile, resultierend aus der langen Halbwertszeit überwunden und gleichzeitig ein therapeutischer Fortschritt erzielt werden.

Die bevorzugten Ausführungsformen haben in der Regel gemeinsam, dass neben 4 -60 mg Propiverin, vorzugsweise 9-45 mg Propiverin, bzw. der äquivalenten Menge eines seiner pharmazeutisch annehmbaren Salze gegebenenfalls eine saure Substanz mit einem $pK_a$-Wert von kleiner 6,65, vorzugsweise mit einem von 1,8 bis 6,5, enthalten ist, und diese beiden Komponenten mit einer oder mehreren retardierenden Schichten, umfassend ein magensaftunlösliches und darmsaftunlösliches und/oder ein magensaftunlösliches und darmsaftlösliches Material, überzogen sind und/oder in eine retardierende Matrix eingebettet sind, die ein quellbares oder unlösliches Material enthält und diese eventuell mit einem magensaftunlöslichen und darmsaftlöslichen bzw. unlöslichen Material überzogen sein kann.

Erfindungsgemäße orale Darreichungsformen sind jedoch prinzipiell auch ohne den Zusatz einer sauren Substanz realisierbar.

Bevorzugte, erfindungsgemäße Zusammensetzungen enthalten neben dem Wirkstoff mindestens eine pharmazeutisch annehmbare organische oder anorganische Säure mit einem $pK_a$-Wert von kleiner 6,65, insbesondere organische Genußsäuren und pharmazeutisch annehmbare Salze mehrbasischer Säuren, beispielsweise Zitronensäure, Weinsäure, Bernsteinsäure, Adipinsäure, Ascorbinsäure, Fumarsäure, Natrium- oder Kaliumhydrogentartrat, Natrium- oder Kalium-dihydrogencitrat, Dinatrium- oder Dikaliumhydrogencitrat usw. oder Gemische dieser Säuren und Salze in einem Verhältnis von 2 : 1 bis 20 : 1, vorzugsweise von 3 : 1 bis 10 :1, bezogen auf das Äquivalentstoffmengenverhältnis zwischen der Gesamtmenge an einwertig saurer Substanz zu Propiverin oder Propiverin-Salz.

Der Säurezusatz dient hierbei nicht der pH-kontrollierten Freisetzung, sondern realisiert durch die Bildung eines "Quasi-Ionenpaars" von Propiverin und Säure die pH-unabhängige Löslichkeit von Propiverin und seinen Salzen und somit eine ausreichende Freisetzung im gesamten Darmtrakt, unabhängig von der Art des vorliegenden Propiverinsalzes, beispielsweise auch von Salzen starker Säuren, wie dem Hydrochlorid. Durch den Säurezusatz wird somit eine übersättigte- bzw. konzentriert-wäßrige Wirkstofflösung von Propiverin, die bei pH-Werten von größer 7 zum sofortigen Ausfall der unlöslichen Propiverinbase führt, verhindert. Im weiteren bewirkt das gebildete Ionenpaar durch seinen Diffusionsdruck eine optimale Freisetzung aus retardierten Teilchen, durch Protonierung einen zusätzlichen "Schutz" des Propiverins vor zu starker N-Oxidation und wahrscheinlich ein verbessertes Resorptionsverhalten des sich physiologisch einstellenden Gleichgewichts zwischen resorbierbarer, freier Propiverinbase und schlechter resorbierbarer protonierter Form, was aus den geringeren Standardabweichungen der pharmakokinetischen Daten der erfindungsgemäßen Formen im Vergleich zum schnellfreisetzenden Handelspräparat ersichtlich ist.

Die pH-abhängig oder pH-unabhängig retardierenden, filmbildenden, matrixbildenden oder in freisetzungsmodifizierenden Systemen verwendeten Materialien sind dem Fachmann allgemein bekannt und im Handel erhältlich, z. B.:

- Polymere und Copolymere von Acryl- und/oder Methacrylsäureestern, wie Eudragit®, von Vinylacetaten und Vinylpyrrolidonen, wie Kollidon® VA64
- Celluloseether und Celluloseester, wie Methocel® und Aquacoat® und Tylose®
- Alginate, wie Kelacid®, Texamid®
- Xanthane, wie Keltrol®, Rhodigel®, weitere Polysaccharide oder modifizierte Polysaccharide, wie Chitosan, Guargummi und Gummi arabicum

- Polyvinylalkohole, wie Mowiol®
- Celluloseacetatphthalate, wie Aquateric®
- Mono-, Di- und Triglyceride, wie Cutina®
- Wachse, wie Montanglycolwachs - HOECHST oder Harze, wie Schellack
- Eiweiße und modifizierte Eiweiße

Die erfindungsgemäße Verwendung von sauren Hilfsstoffen in Kombination mit retardierenden Überzügen und/oder einer retardierenden Matrix gestattet sowohl die Herstellung von sogenannten Multiple-Unit-Formulierungen als auch von sogenannten Single-Unit-Formulierungen.

Eine besonders bevorzugte Ausführungsform stellen sogenannte Multiple-Unit-Formulierungen in Form von Pellets dar.

Diese Teilchen können erfindungsgemäß eine Korngröße von 0,1 mm bis 2,5 mm, bevorzugt 0,6 bis 1,5 mm, aufweisen und anschließend in Kapseln oder Sachets abgefüllt werden oder zusammen mit Tablettierhilfsstoffen zu Tabletten verpresst werden, wobei diese sphäroiden Teilchen in gleicher oder verschiedener Größe in einer Formulierung enthalten sein können.

Durch Kombination mehrerer Technologieschritte werden zuerst in an sich bekannter Art und Weise sphäroide Teilchen saurer Substanzen mit einem Durchmesser von 0,1 bis 2,0 mm, vorzugsweise von 0,5 bis 1,2 mm, beispielsweise Teilchen kristalliner Zitronensäure, Weinsäure usw., im Wirbelschichtverfahren mit Suspensionen, bestehend aus Hilfsstoffen, wie Lactose, aus gelösten Säuren, wie z. B. Zitronensäure, aus Bindemitteln, wie z. B. Polyvinylpyrrolidon, und aus Antiklebemitteln, wie z. B. Talkum, ausgerundet.

Um die Auflösung der in Wasser hochlöslichen Genußsäuren oder der anderen, sauren Substanzen zu steuern, werden erfindungsgemäß die ausgerundeten Säurekerne beispielsweise in der Wirbelschicht mit einer Lackschicht versehen.

Die Materialien der magensaftunlöslichen und pH-abhängig darmsaftlöslichen Membranen wie Eudragit® S und Eudragit® L sind allgemein üblich. Analoges gilt für den eventuellen Zusatz von Bindemitteln oder Weichmachern, wie Polyvinylpyrrolidon oder Triethylcitrat.

Nicht vorhersehbar war, dass im Falle von Propiverin oder seinen Salzen eine solche Lackschicht für die erfindungsgemäßen Formen äußerst vorteilhaft ist. Beim Fehlen einer solchen kann die durch einen hohen Diffusionsdruck bedingte und ungehinderte Diffusion trotz Retardierung der wirkstoffhaltigen Teilchen und der nachfolgend magensaftresistenten Retardierung dieser Teilchen zu einer unerwünschten schnellen Freisetzung von Propiverin und durch eine vorzeitige Erschöpfung des Säurereservoirs im weiteren Freisetzungsverhalten zu einer deutlich verminderten Menge an freigesetztem Propiverin führen (Ausführungsbeispiel 7).

In an sich bekannter Art und Weise wird im nächsten technologischen Schritt, beispielsweise in der Wirbelschicht, Propiverin oder eines seiner Salze unter Zusatz von Hilfsstoffen, Kleb- und Bindemitteln und weiteren Anteilen von den bereits erwähnten Säuren oder sauren Substanzen als alkoholischwäßrige Suspension in der erforderlichen Menge aufgetragen. Es besteht im weiteren auch die Möglichkeit, mikronisierten Wirkstoff mit und ohne Zusatz von mikrokristallinen Hilfsstoffen und den erwähnten Säuren oder sauren Substanzen nach vorhergehender Übersprühung der retardierten Säurekerne mit einer Klebstofflösung in Pulverform aufzutragen. Die Zugabe von Anteilen gleicher oder verschiedenartiger Säuren im Verhältnis zu der im lackierten Säurekern befindlichen Säure ist vorteilhaft, um durch Abpufferung des durch die nachfolgende Retardschicht eindringlichen Darmsaftes eine Initiallösung/freisetzung zu erhalten.

Für die erfindungsgemäßen Zusammensetzungen mit einer verzögerten und bis zu deren Ende kontrollierten Freisetzung ist es günstig, auf den mit Wirkstoff beladenen, retardierten Säurekern im nächsten technologischen Schritt eine oder mehrere Retardschichten einzeln oder als gemeinsames Gemisch mit und ohne weiteren Zusatz von Bindemitteln und Hilfsstoffen aufzutragen.

Die erfindungsgemäß zu verwendenden magensaftunlöslichen, pH-unabhängig darmsaftunlöslichen Materialien unterschiedlich hoher Permeabilität und die magensaftunlöslichen, pH-abhängig darmsaftlöslichen Materialien sind im Handel erhältlich, z. B. Eudragit® RS, Eudragit® RL und Eudragit® S bzw. Eudragit® L.

Die optimalen Mengen der einzelnen Bestandteile, bzw. die Zusammensetzung der entsprechenden Gemische hängen von verschiedenen Faktoren, wie z. B. dem gewünschten Retardierungseffekt, der Art der einzelnen Retardierungsbestandteile mit deren spezifischer Löslichkeit und Permeabilität und dem Verhältnis von saurer Substanz zum Wirkstoff Propiverin ab. Diese Parameter variieren dabei vollkommen unabhängig voneinander und haben eine wesentliche Bedeutung für die kontrollierte und langanhaltende Freisetzung des Propiverins.

Mittels aufwendiger in vitro- und in vivo-Versuche unter Einbeziehung einer gefundenen in vivo-/in vitro-Korrelation konnten diese Schwierigkeiten gelöst werden. Durch die gefundene in vivo-/in vitro-Korrelation ist es möglich, ohne aufwendige, klinische in vivo-Versuche, erfindungsgemäße Darreichungsformen von Propiverin jedweder Zusammensetzung durch eine einfache Bestimmung von in vitro-Freisetzungswerten herzustellen. Entsprechen die ermittelten Freisetzungswerte den gefundenen Freisetzungsbereichen, so ist diese Darreichungsform klinisch relevant.

Es wurde unerwarteterweise gefunden, dass es günstig ist, wenn sich die auf die wirkstoffhaltigen Säurekerne aufgesprühte Retardschicht im resorptionsfähigen Teil des Magen- und Darmtraktes im Falle von Propiverin solange nicht auflöst, bis praktisch der gesamte Wirkstoff in Form seines Säure-Ionenpaars zeitkontrolliert herausdiffundiert ist. Vorteilhafterweise sollte diese Hülle die im Kern befindliche Säure solange zurückhalten, bis die Bildung des Propiverin-Säure-Ionenpaars vollständig abgeschlossen ist. Geht die Hülle vorzeitig in Lösung bzw. wird sie zu undicht, so dringt der stets in großem Überschuß vorhandene Darmsaft in das Innere der Teilchen ein und neutralisiert die dort vorhandene Säure. Damit kann wegen der geringen Löslichkeit des Propiverins im pH-Bereich des Darmsaftes praktisch kaum noch Wirkstoff in Lösung gehen und aus den Teilchen herausdiffundieren. Die im Inneren der Teilchen vorhanden Säure erniedrigt den pH-Wert der eindringenden Darmflüssigkeit und bildet im weiteren das entsprechende Ionenpaar. Danach diffundiert die im Inneren entstehende Lösung des Propiverin-Säure-Ionenpaars über die Membran in den Darm. Obwohl hier wieder ungünstige pH-Verhältnisse herrschen, treten offensichtlich Übersättigungsphänomene auf, die eine gute Resorption des an sich kaum löslichen Wirkstoffes sicherstellen. Selbstverständlich reduziert sich die in den Teilchen vorhanden Säure im Laufe der Freigabe immer mehr, so dass an sich eine starke Abnahme der diffundierenden Menge bzw. der Freisetzung von Propiverin eintreten müßte. Um letzteres zu verhindern, sollte die Retardschicht, die die Freisetzungsgeschwindigkeit steuert, vorzugsweise im Darmsaft "teillöslich" sein. Das Wort "teillöslich" ist dabei im Sinne einer bestimmten Permeabilität bzw. als Diffusionswiderstand zu verstehen. Diese Retardschicht soll im weiteren zusätzlich garantieren, dass im Innern der Teilchen stets saure pH-Verhältnisse herrschen und eventuell durch Nahrungsmitteleinflüsse oder durch andere Einflüsse auftretende pH-Schwankungen ausgeglichen werden.

Bereits geringe Abweichungen im Verhältnis von Eudragit® RS/Eudragit® RL/ Eudragit® S führen zu drastischen Veränderungen der Freisetzungsprofile. Wird das gefundene Verhältnis Eudragit® RS/ Eudragit® RL/Eudragit® S von 2:1:2 geringfügig auf ein Verhältnis von 1,5:1:2,5 verändert (Beispiel 9), so führt diese Verringerung des darmsaftunlöslichen Bestandteiles mit geringerer Permeabilität (Eudragit® RS) und der erhöhte Anteil am darmsaftlöslichen Material (Eudragit® S) zu einer zu schnellen Wirkstofffreisetzung bzw. zu einer zu starken Diffusion des sauren Bestandteiles.

Der Effekt ist wie folgt erklärbar: Da sich das magensaftunlösliche, darmsaftlösliche Material dieser Schicht nach einer gewissen Aufenthaltszeit der Teilchen im Darmsaft herauslöst, wird effektiv bei dem Verhältnis Eudragit® RS/Eudragit® L/Eudragit® S von 2:1:2 eine zu starke Freisetzung im oberen Darmabschnitt unterdrückt und insgesamt die Wirkstofffreisetzung in mittlere und tiefe Darmabschnitte verlagert. Diese Retardschicht reduziert somit die insbesonders rasche Resorption im oberen Darmabschnitt, ohne die Gesamtfreigabe des Wirkstoffes aus den Teilchen zu reduzieren. Dies führt eindeutig zu einer Verlängerung der Wirkstofffreisetzung.

Um eine Magensaftresistenz und im weiteren nicht zu hohe Anfangsfreisetzungswerte zu erreichen bzw. die Freisetzungscharakteristik weiter zu modifizieren, ist allgemeiner Fachstandard, weitere Retardierungsschichten mit magensaftunlöslichen und darmsaftlöslichen Materialien, beispielsweise Eudragit® L bzw. Eudragit® S, aufzubringen.

Im weiteren können die erfindungsgemäß verwendbaren Retardierungsschichten übliche Hilfsstoffe enthalten, wie beispielsweise Weichmacher, Netzmittel und Antiklebemittel. Beispiele geeigneter, pharmakologisch unbedenklicher Weichmacher sind Glycerintriacetat, Polyethylenglykole und Zitronensäureester, wie Triethylcitrat. Das Aufbringen der Retardierungsschichten auf die Wirkstoff enthaltenden Teilchen kann nach an sich bekannten Methoden, beispielsweise in einem rasch rotierenden Kessel oder im Wirbelschichtverfahren durch Aufsprühen der Lacke erfolgen. Die anschließende Trocknung der Pellets, um die durch die Suspension enthaltenen Restlösungsmittel zu entfernen, ist Stand der Technik.

Die erhaltenen retardierten Teilchen, die in Form von retardierten Pellets, Granulat- oder Kompaktatteilchen vorliegen können, können gewünschtenfalls in Kapseln oder Sachets, vorzugsweise Hartgelatine-Kapseln, abgefüllt werden. Dabei ist es möglich, Teilchen verschiedener Verzögerungsstufen zu mischen und gegebenenfalls auch unverzögerte Wirkstoffteilchen als sogenannte Startdosis hinzuzufügen. Die Retardteilchen können aber auch mit Tablettierhilfsstoffen, wie Cellulose, Lactose, Magnesiumstearat und dergleichen, zu Tabletten verpreßt werden. Dies ist insbesondere bei Retardteilchen mit einem Durchmesser unter 1 mm ohne nennenswerte Beschädigung der Retardierungsschichten möglich. Eine solche Tablette zerfällt in weniger als 1 Minute und gibt - wie die Hartgelatine-Kapsel - die Propiverin-Retardteilchen in erfindungsgemäßer Form frei.

Eine weitere bevorzugte Ausführungsform sogenannter Multiple-Unit-Formulierungen stellen Granulate und Kompaktteilchen dar, die neben Propiverin oder einem seiner Salze eine oder mehrere saure Substanzen enthalten, die nicht in einer retardierenden Matrix eingebettet sind, sondern die lediglich dieses Gemisch mit einem oder mehreren freisetzungsverzögerten Überzügen beinhalten, welche anschließend zu Tabletten verpreßt werden.

Diese sogenannten Sphäroidtablettenfonmulierungen werden hergestellt, indem Propiverin oder eines seiner pharmazeutisch verwendbaren Salze im erfindungsgemäßen Molverhältnis mit einer oder mehreren sauren Substanzen mit Sphäronisierungsmitteln, wie z. B. Lactose, mikrokristalliner Cellulose, Hydroxypropylcellulose, mit Gleitmitteln, wie z. B. Magnesiumstearat, und mit weiteren Hilfsmitteln, wie z. B. Polyvinylpyrrolidon, in mikrokristalliner Form, z. B. in einer Korngröße von kleiner 0,25 mm, unter starkem Druck kompaktiert, im weiteren erneut gebrochen und z. B. auf eine Korngröße von 0,5 - 1,5 mm gesiebt, der Feinanteil erneut kompaktiert und diese Technologieschritte solange wiederholt werden, bis die gesamte Mischung der Granulatteilchen in die gewünschte Größe überführt ist.

Derartige Granulatteilchen können neben der beschriebenen Kompaktierung aber auch durch andere Verfahren, beispielsweise durch Extrusion/Sphäronisation, hergestellt werden.

Im weiteren werden die Granulatteilchen mit allgemein bekannten magensaftunlöslichen und darmsaftlöslichen und/oder magensaftunlöslichen und darmsaftunlöslichen Retardierungsmitteln, wie beispielsweise Eudragit® NE, Eudragit® L usw. überzogen. Allgemein bekannte Tablettierhilfsmittel, wie mikrokristalline Cellulose, Crospovidon, Polyvinylpyrrolidon, Magnesiumstearat usw. werden zugegeben, die gesamte Mischung innig gemischt und zu Tabletten verpreßt. Im weiteren können die so hergestellten Tabletten mit geeigneten Überzügen, die freisetzungsmodifizierend sein können; überzogen sein. Die Retardierungsschichten garantieren auch in diesem Falle die Bildung eines Propiverin-Säure-Ionenpaars und dessen kontrollierte Diffusion. Da die erfindungsgemäßen Formulierungen in weniger als 5 Minuten zerfallen und dabei hunderte von retardierten Propiverin-Säure-Teilchen freisetzen, hat die Zerfallszeit auf das Freisetzungsverhalten keinen Einfluss.

Im Gegensatz zu den sogenannten, auf Pellets oder sphäroiden Teilchen basierenden Multiple-Unit-Formulierungen können Retardpräparate von Propiverin aber grundsätzlich auch auf andere Art und Weise, z. B. als Single-Unit-Formulierung hergestellt werden.

Insbesondere können geeignete Freisetzungscharakteristiken auch durch Matrixretardierung, beispielsweise mittels einer Matrixtablette, erreicht werden.

Bevorzugt wird aber auch in dieser Ausführungsform eine der bereits erwähnten sauren Substanzen verwendet und sowohl die saure Substanz als auch der Wirkstoff in die retardierende Matrix eingebettet. Hierbei sind grundsätzlich die eingangs genannten sauren Substanzen, Polymere, Dosierungen und Molverhältnisse von saurer Substanz im Verhältnis zu Propiverin geeignet.

Beispielsweise werden zur Herstellung von erfindungsgemäßen Matrixtabletten Propiverin oder eines seiner pharmazeutisch annehmbaren Salze gegebenenfalls mit einer oder mehreren sauren Substanzen im erfindungsgemäßen Molverhältnis mit retardierenden, matrixbildenden Hilfsstoffen, wie beispielsweise Celluloseethern, Celluloseestern, Alginaten, Xanthanen, Polyvinylalkoholen, Fetten, Wachsen und weiteren Tablettierhilfsmitteln, wie beispielsweise Magnesiumstearat, in mikrokristalliner Form mit einer Korngröße von vorzugsweise kleiner als 0,25 mm innig vermischt und zu Tabletten verpreßt. Im Falle von Propiverin und seinen Salzen wurde gefunden, dass dabei die Auswahl der sauren Substanzen unabhängig von ihrer Wasserlöslichkeit erfolgen kann. So führen z. B. die schwerer in Wasser lösliche Adipinsäure und auch die leichter in Wasser lösliche Weinsäure bei erfindungsgemäßer Verwendung zu gleichen Freisetzungsprofilen. Bei Kontakt dieser Tabletten mit Wasser entsteht z. B. im Falle von einer Matrix aus Alkylcellulosen sofort eine hochviskose Gelschicht, die die Diffusion des gebildeten Propiverin-Säure-Ionenpaars entsprechend der gewünschten Freisetzungsrate reduziert.

Neben den bereits beschriebenen Darreichungsformen, bestehend aus Propiverin, einer oder mehreren sauren Substanzen und retardierenden Materialien, besteht auch die Möglichkeit, Kombinationen von Propiverin oder eines oder mehrerer seiner pharmazeutisch annehmbaren Salze mit einem oder mehreren anderen Wirkstoffen in gleicher Art und Weise zu retardieren, unabhängig davon, ob die verwendeten Kombinationswirkstoffe saure Substanzen darstellen, wie im Falle der bereits erwähnten Ascorbinsäure, oder nicht.

Anhand folgender Beispiele wird die Erfindung näher erläutert, ohne auf sie beschränkt zu sein.

## Beispiele

Alle Prozentangaben beziehen sich, sofern nicht anders angegeben, auf Gewichtsprozent (Gew.-%).

### Beispiel 1 (Vergleich)

### Pelletformulierung mit Propiverinhydrochiorid - 100%ige Freisetzung nach 15 min

Zur Herstellung sphärischer Zitronensäurekerne werden 3,5 kg Zitronensäure mit einer Teilchengröße von 0,7 -1,0 mm (Roche) bei einer Lufttemperatur von 45-°C mittels 2-Stoffdüsen in der Wirbelschicht mit einer isopropanolisch-wäßrigen Suspension, bestehend aus 105 g Polyvinylpyrrolidon (Kollidon® K25), 35 g Zitronensäure, 275 g Lactose (Microtose®), 210 g Talk, 1500 g 2-Propanol und 300 g Wasser besprüht und eine Ausbeute von 4,060 kg (98,4 % der Theorie, bezogen auf lösungsmittelfreies Material) an gerundeten Zitronensäurepellets erhalten.

3,75 kg dieser gerundeten Zitronensäurepellets werden im nächsten Technologieschritt mit einer isopropanolisch-wäßrigen Suspension, bestehend aus 600 g Eudragit® S12,5 (entsprechend 75 g Eudragit® S), 600 g Eudragit® L 12,5 (entsprechend 75 g Eudragit® L), 20 g Triethylcitrat, 100 g Talk, 1500 g 2-Propanol und 300 g Wasser in analoger Weise retardiert. Die Ausbeute betrug 4,013 kg (99,8 % der Theorie, bezogen auf lösungsmittelfreies Material).

Zur Auftragung des Wirkstoffes werden 3,95 kg der retardierten Zitronensäurekerne in analoger Weise mittels 2-Stoffdüsen in der Wirbelschicht bei 45 °C Zulufttemperatur mit einer isopropanolisch-wäßrigen Suspension von 1300 g mikronisiertem Propiverinhydrochlorid, 280 g Polyvinylpyrrolidon (Kollidon® K25), 50 g Zitronensäure, 200 g Talk, 50 g Magnesiumstearat, 2100 g 2-Propanol und 400 g Wasser besprüht. Als Ausbeute wurden 5,772 kg an wirkstoffhaltigen, zuvor retardierten Zitronenpellets erhalten (90 %, bezogen auf lösungsmittelfreies Material).

Der Gehalt von Propiverinhydrochlorid in den Pellets wurde mit Hilfe der im Beispiel 6 beschriebenen Methode zu 20,9 %, der Gehalt an Zitronensäure zu 59,8 % bestimmt. Daraus ergibt sich ein molares Verhältnis von Propiverinhydrochlorid zu Zitronensäure von 1 : 6,0.
Aus den eingesetzten Substanzmengen berechnet sich ein Molverhältnis Propiverinhydrochlorid/Zitronensäure von 1 : 5,2. Diese Differenz ist durch Sprüh- und Abriebverluste zu erklären.

Zur Abfüllung werden 2000 g der erhaltenen Pellets mit 10g mikrofeinem Talk versetzt und 15 min gemischt. Die Freisetzungswerte wurden mittels der in Beispiel 5 beschriebenen Methode bestimmt und sind in der dort dargestellten Tabelle dokumentiert.

Anschließend wird gesiebt und die Fraktion mit einer Korngröße von 0,7 - 1,25 mm Durchmesser (98,0 %) weiterverarbeitet. 215 mg Pellets entsprechend 45 mg Propiverinhydrochlorid werden in Hartgelatinekapseln abgefüllt und für die Bioverfügbarkeitsstudie (Beispiel 13) verwendet.

**Beispiel 2 (Vergleich)**

**Pelletformulierung mit Propiverinhydrochlorid - ca. 50 %ige Freisetzung nach 3 Stunden**

Zur Herstellung sphärischer Zitronensäure-Starterkerne werden 3500 g Zitronensäure Granulat (Roche) mit einer Teilchengröße von 0,7-1,0 mm in technisch gleicher Art und Weise, wie in Beispiel 1 beschrieben, mit einer Suspension aus 30 g Polyvinylpyrrolidon (Kollidon®), 10 g Zitronensäure, 78 g Lactose (Microse®) und 60 g Talk in 340 g 2-Propanol und 75 g Wasser in der Wirbelschicht besprüht. Es wurden 3665 g gerundete Zitronensäurepellets erhalten (99,7 % der Theorie bezogen auf die eingesetzte Trockensubstanz).

3660 g der oben beschriebenen Zitronensäurepellets werden im nächsten Schritt mit einer Suspension von 600 g Eudragit® S 12,5 (75 g Trockensubstanz Eudragit® S), 600 g Eudragit L 12,5 (75 g Trockensubstanz Eudragit® L), 20 g Triethylcitrat und 100 g Talk, mikrofein, in 1500 g 2-Propanol und 300 g Wasser besprüht. Die Ausbeute betrug 3930 g, entsprechend 100 %, bezogen auf lösungsmittelfreies Material.

3650 g dieser Pellets wurden mit in oben beschriebener Art und Weise mit einer Suspension von 1000 g mikronisiertem Propiverinhydrochlorid, 215 g Polyvinylpyrrolidon (Kollidon® K25), 40 g Zitronensäure, 155 g Talk, mikrofein und 40 g Magnesiumstearat in 2100 g 2-Propanol und 310 g demineralisiertem Wasser besprüht. Als Ausbeute wurden 5030 g Wirkstoffpellets erhalten. Das entspricht einer Ausbeute von 98,6 %, bezogen auf die Trockenmasse.

3500 g dieser Wirkstoffpellets werden im nächsten Technologieschritt in der Wirbelschicht bei einer Zulufttemperatur von 40 - 45.°C mittels 2-Stoffdüsen mit einer isopropanolisch-wäßrigen Suspension von 420 g Eudragit® RS 12,5 (entsprechend 52,5 g Eudragit® RS Trockenmasse), 420 g Eudragit® RL 12,5 (entsprechend 48,4 g Eudragit RLTrockenmasse), 560 g Eudragit® S 12,5 (entsprechend 75 g Eudragit® S Trockenmasse), 20 g Triethylcitrat, 120 g Talkum, 1400 g 2-Propanol und 210 g Wasser retardiert und in der Wirbelschicht intensiv getrocknet. Als Ausbeute wurden 3815 g (100 % der Theorie, bezogen auf lösungsmittelfreies Material) erhalten.

Auf 2900 g der retardierten Wirkstoffpellets wird im nächsten Schritt unter den bereits oben geschilderten Bedingungen eine Suspension von 1392 g Eudragit® L 12,5, 16 g Triethylcitrat und 105 g mikrofeiner Talk in 1135 g 2-Propanol und 380 g Wasser aufgetragen. Die Auswaage betrug 3190 g Pellets (99,8 % der theoretischen Ausbeute, bezogen auf die Trockenmasse).

Zur weiteren Retardierung der Pellets wird anschließend eine Suspension von 300 g Eudragit® RL 12,5 (37,5 g Trockenmasse), 4 g Triethylcitrat, 35 g mikrofeinem Talk und 96,5 g Magnesiumstearat in 1370 g 2-Propanol und 340 g Wasser auf 2500 g der retardierten Wirkstoffpellets aufgetragen. Die Auswaage betrug 2583 g an Pellets, was einer Ausbeute von 99,5 % der Theorie, bezogen auf die Trockenmasse, entspricht.

Vor der Abfüllung wurden 2500 g Pellets mit 12,5 g mikrofeinem Talk 15 min gemischt und anschließend gesiebt. 2425 g (96,5 %) der Pellets weisen eine Korngröße von 0,7 - 1,25 mm Durchmesser auf.

Der Gehalt an Propiverinhydrochlorid in den Pellets wurde gemäß der im Beispiel 6 beschriebenen Methode zu 13,7 % bestimmt.

Der Gehalt an Zitronensäure in den vorliegenden Pellets wurde ebenfalls mit der im Beispiel 6 beschriebenen Methode zu 53,0 % bestimmt. Daraus ergibt sich ein molares Propiverinhydrochlorid-Zitronensäure-Verhältnis von 1 : 8,1.

Aus den eingesetzten Stoffmengen wurde ein molares Verhältnis Propiverinhydrochlorid zu Zitronensäure von 1 : 6,9 errechnet. Auch diese Differenz ist durch Sprüh- und Abriebverluste zu erklären.

Für die Bioverfügbarkeitsstudie des Beispiels 13 werden entsprechend 45 mg Propiverinhydrochlorid 328 mg Pellets in Hartgelatinekapseln abgefüllt. Die Freisetzungswerte wurden mittels der im Beispiel 5 beschriebenen Methode bestimmt und sind in der dort dargestellten Tabelle wiedergegeben.

**Beispiel 3**

**Pelletformulierung mit Propiverinhydrochlorid - ca. 20%ige Freisetzung nach 3 Stunden und ca. 80%ige Freisetzung nach 10 Stunden Beispiel 3.1: Ansatzgröße im Technikumsmaßstab**

In gleicher Art und Weise, gleicher Ansatzgröße und in gleicher stofflicher Zusammensetzung, wie im Beispiel 1 beschrieben, werden erneut 5638 g (96,7 % der Theorie, bezogen auf lösungsmittelfreies Material) an propiverinhydrochloridhaltigen, zuvor retardierten Zitronensäurepellets erhalten.

2900 g der oben beschriebenen Wirkstoffpellets werden mit einer Suspension von 600 g Eudragit® RS 12,5 (75 g Trockensubstanz Eudragit® RS), 304 g Eudragit® RL 12,5 (38 g Trockensubstanz Eudragit® RL), 600 g Eudragit® S 12,5 (75 g Trockensubstanz Eudragit® S), 20 g Triethylcitrat und 120 g mikrofeinem Talk in 1415 g 2-Propanol und 220 g demineralisiertem Wasser besprüht. Die Auswaage betrug 3227 g retardierte Wirkstoffpellets (entsprechend 100 % theoretische Ausbeute, bezogen auf lösungsmittelfreies Material).

3100 g dieser Pellets werden anschließend mit einer Suspension bestehend aus 868 g Eudragit® S 12,5 (108,5 g Eudragit S Trockenmasse), 11 g Triethylcitrat, 65 g mikrofeinem Talkum, 840 g 2-Propanol und 100 g Wasser besprüht. Die Auswaage an Pellets betrug 3285 g, das entspricht 100 % der Theorie, bezogen auf lösungsmittelfreie Pellets.

Vor der Abfüllung wurden 3200 g der so erhaltenen Pellets unter Zugabe von 16 g mikrofeinem Talk 15 Minuten gemischt und anschließend gesiebt. Die Komgrößenfraktion 0,7 mm bis 1,25 mm (3120 g, entsprechend 97 % der Theorie) weist einen experimentell bestimmten Gehalt von 18,8 % an Propiverinhydrochlorid auf.

Der Gehalt an Zitronensäure in den Pellets wurde mit Hilfe der im Beispiel 6 beschriebenen potentiometrischen Titration zu 50,7 % bestimmt.

Aus den experimentell ermittelten Gehalten ergibt sich ein molares Wirkstoff Zitronensäure-Verhältnis von 1 : 5,7.

Das theoretische Verhältnis, berechnet auf der Grundlage der eingesetzten Massen, beträgt 1 : 5,2. Die Differenz ist ebenfalls durch Sprüh- und Abriebverluste zu erklären.

Entsprechend 45 mg Propiverinhydrochlorid wurden 240 mg der oben beschriebenen Pellets in Hartgelatinekapseln abgefüllt und für die Bioverfügbarkeitsstudie des Beispiels 13 verwendet. Die Freisetzungswerte wurden mit Hilfe der im Beispiel 5 beschriebenen Methode ermittelt und sind in der dort dargestellten Tabelle aufgeführt.

**Beispiel 3.2: Ansatzgröße im produktionsrelevanten Maßstab**

Zur Herstellung sphärischer Zitronensäurekerne als Starterpellets wurden 250,0 kg Zitronensäuregranulat (Roche) mit einer Teilchengröße zwischen 0,7 mm und 1,0 mm mittels 2-Stoffdüsen in der Wirbelschicht mit einer Suspension von 7,5 kg Polyvinylpyrrolidon (Kollidon® K25), 2,5 kg Zitronensäure, 19,6 kg Lactose und 15,0 kg Talk, mikrofein in 89,3 kg 2-Propanol und 19,6 kg demineralisiertem Wasser besprüht. Auf diese Weise wurden 282,0 kg sphärischer Zitronensäure-Starterkerne erhalten (95,7 % der Theorie, bezogen auf ein lösungsmittelfreies Material).

200,0 kg der Starterkerne wurden anschließend in technisch gleicher Art und Weise mit einer Suspension von 4,0 kg Eudragit® S 140, 4,0 kg Eudragit L100, 1,1 kg Triethylcitrat und 5,3 kg mikrofeinem Talkum in 94,0 kg 2-Propanol und 11,0 kg demineralisiertem Wasser besprüht. Die Auswaage betrug 214,0 kg, das entspricht 99,8 % der Theorie, bezogen auf die eingesetzte Trockenmasse.

Durch eine anschließende Siebung wurden alle Pellets mit einem Durchmesser von kleiner als 1,25 mm isoliert.

Die so erhaltenen retardierten Starterkerne wurden im folgenden Technologieschritt in zwei Stufen mit einer Suspension von insgesamt 85,3 kg Propiverinhydrochlorid, 22,7 kg Polyvinylpyrrolidon (Kollidon® K25), 8,5 kg Zitronensäure, 11,1 kg mikrofeinem Talk, 0,802 kg Magnesiumstearat, 165,5 kg 2-Propanol und 48,2 kg demineralisiertem Wasser besprüht. Die Ausbeute über beide Stufen betrug 94,8 %, bezogen auf die eingesetzte Trockenmasse.

243,0 kg der so erhaltenen Wirkstoffpellets mit einem Gehalt von 21,75 % Propiverinhydrochlorid wurden zur Retardierung in der Wirbelschicht mit einer Suspension von 54,7 kg Eudragit® RS 12,5 (6,7 kg Eudragit® RS Trockensubstanz), 27,8 kg Eudragit RL (3,4 kg Eudragit RL Trockensubstanz), 6,7 kg Eudragit® S100, 1,8 kg Triethylcitrat und 10,8 kg Talk, mikrofein in 207,3 kg 2-Propanol und 23,8 kg demineralisiertem Wasser überzogen. Die Ausbeute betrug 99,2 % der Theorie an lösungsmittelfreien Retardteilchen.

237,4 kg der oben beschriebenen retardierten Wirkstoffpellets wurden in der Wirbelschicht mit einer Suspension von 5,7 kg Eudragit® S100, 0,582 kg Triethylcitrat und 3,5 kg Talkum, mikrofein in 44,6 kg 2-Propanol und 5,4 kg demineralisiertem Wasser überzogen.

Die Pellets wurden vor dem Abfüllen 60 h bei 70 °C getrocknet. 13,0 kg der Pellets wurden anschließend mit 65 g Talkum 10 min gemischt und danach durch ein 1,25 mm Sieb gesiebt. 12,8 kg dieser Pelletfraktion mit einer Teilchengröße von kleiner als 1,25 mm wies einen mittels der im Beispiel 6 beschriebenen Methoden bestimmten Gehalt von 18,8 % an Propiverinhydrochlorid und 49,8 % an Zitronensäure auf. Das molare Wirkstoff-Zitronensäure-Verhältnis beträgt 1 : 5,6.

Jeweils 240 mg der so erhaltenen Pellets, entsprechend 45 mg Propiverinhydrochlorid, wurden in Hartgelatinekapseln abgefüllt und für die Bioäquivalenzstudie des Beispiels 14 verwendet.

Die Freisetzung von Propiverin aus den Pellets wurde unter den im Beispiel 5 beschriebenen Bedingungen durchgeführt. Die Ergebnisse sind in der dort dargestellten Tabelle aufgeführt.

**Beispiel 4**

**Pelletformulierung mit Propiverin**

2400 g sphärischer Zitronensäurekerne, die in gleicher Art und Weise hergestellt wurden, wie bereits im Beispiel 3.2 beschrieben, wurden in der Wirbelschicht bei einer Zulufttemperatur von 40 - 75 °C mit einer Suspension, bestehend aus 48 g Eudragit® S 100, 48 g Eudragit L 100, 13 g Triethylcitrat, 65 g mikrofeinem Talkum, 1860 g Isopropanol und 200 g Wasser überzogen.

2500 g der so erhaltenen retardierten Starterkerne wurden unter den gleichen technischen Bedingungen mit einer Suspension von 828 g Propiverin 177 g Polyvinylpyrrolidon (Kollidon® K25), 63 g Zitronensäure, 200 g Talkum, mikrofein und 32 g Magnesiumstearat in 3100 g 2-Propanol und 400 g Wasser besprüht. Die Auswaage betrug 3740 g Wirkstoffpellets, entsprechend 98,4 % der Theorie, bezogen auf die eingesetzte Trockensubstanz.

3250 g der Wirkstoffpellets wurden in gleicher Art und Weise mit einer Suspension von 720 g Eudragit® RS 12,5 (90 g Trockenmasse Eudragit® RS), 368 g Eudragit® RL 12,5 (46 g Eudragit® RL Trockenmasse), 90 g Eudragit® S 100, 24 g Triethylcitrat und 146 g Talkum, mikrofein, in 1930 g 2-Propanol und 300 g demineralisiertem Wasser besprüht.

3500 g der so erhaltenen, retardierten Wirkstoffpellets wurden in der Wirbelschicht unter identischen Bedingungen mit einer Suspension, bestehend aus 86 g Eudragit® S 100, 9 g Triethylcitrat, 52 g mikrofeinem Talkum, 129,9 g 2-Propanol und 80 g Wasser besprüht. Die so erhaltenen Pellets wiesen ein mittels der im Beispiel 6 beschriebenen Methoden bestimmten Gehalt von 19,4 % Propiverinhydrochlorid sowie einen Gehalt von 40,4 % an Zitronensäure auf. Daraus ergibt sich ein molares Wirkstoff-Zitronensäure-Verhältnis von 1 : 4,0.

Die Freisetzung von Propiverin aus den Pellets wurde entsprechend der im Beispiel 5 beschriebenen Methode bestimmt und ist in der dort dargestellten Tabelle wiedergegeben.

**Beispiel 5,**

**Bestimmung der Freisetzungswerte - Gegenüberstellung der Freisetzungswerte der Beispiele 1 - 4, 7 - 13**

Die Bestimmung der Freisetzung von Propiverinhydrochlorid bzw. Propiverin aus allen beschriebenen oralen Darreichungsformen erfolgt mit Hilfe der in der Ph. Eur. 3, 2.93 beschriebenen Drehkörbchen-Apparatur bei 100 Upm über 17 Stunden.

Dazu wird jeweils eine 45 mg Propiverinhydrochlorid entsprechende Menge Pellets in 6 Drehkörbchen eingewogen. Die Freisetzung erfolgt zunächst eine Stunde in 750 ml Magensaftmedium (0,1 M Salzsäurelösung) bei 37 °C. Dieses Medium wird nach Messung des 1-Stunden-Wertes verworfen und die Freisetzung in 750 ml eines 50 mM KaliumdihydrogenphosphatPuffers pH 5,8 bei 37 °C für weitere 16 Stunden durchgeführt.

Die Quantifizierung von Propiverinhydrochlorid bzw. Propiverin im Freisetzungsmedium erfolgt mit Hilfe eines on line-gekoppelten UV/VIS-Spektralphotometers. Zur Messung wird das Freisetzungsmedium zu definierten Zeiten aus den jeweiligen Freisetzungsbehältem durch Polypropylenfilter über eine 6-Kanal-Schlauchpumpe in die Durchflußküvetten des UV/VIS-Spektralphotometers gepumpt. Die Messung der Extinktion erfolgt bei 239 nm, wobei als Hintergrund zusätzlich die Extinktion bei 247 nm bestimmt wird. Zur Berechnung des Gehaltes von Propiverinhydrochlorid/Propiverin wird der Extinktionswert bei der Hintergrundwellenlänge vom Extinktionswert der Meßwellenlänge abgezogen.

Die Berechnung der Freisetzungswerte erfolgt in Relation zu einer Probe Referenzsubstanz, die unter den gleichen Bedingungen im UV/VIS-Spektrometer vermessen wird. Die Menge an Propiverinhydrochlorid bzw. Propiverin, die während der ersten Stunde in 0,1 M Salzsäure freigesetzt wird, wird zu den weiteren Freisetzungswerten addiert.

Die erhaltenen Freisetzungswerte sind in den Tabellen 1 und 2 als Mittelwerte einer Sechsfachbestimmung enthalten.

**Tabelle 1 : Freisetzung von Propiverinhydrochlorid/Propiverin in Prozent - Beispiele 1- 4 (n.b. - nicht bestimmt)**

| Zeit [h] | Beispiel 1 (Pellet) Propiverinhydrochlorid-100 % nach 15 min | Beispiel 2 (Pellet) Propiverinhydrochlorid-50 % nach 3 h | Beispiel 3.1 (Pellet) Propiverinhydrochlorid-20 % n. 3 h, 80 % n. 10 h | Beispiel 3.2 (Pellet) Propiverinhydrochlorid-20 % n. 3 h, 80 % n. 10 h | Beispiel 4 (Pellet) Propiverin-20%n.3h,70%n. 10 h |
|---|---|---|---|---|---|
| 0,08 | 103,06 | n.b. | n.b. | n.b. | n.b. |
| 0,17 | 101,41 | n.b. | n.b. | n.b. | n.b. |
| 0,25 | 100,05 | n.b. | n.b. | n.b. | n.b. |
| 0,5 | n.b. | 0,43 | 0,00 | 0,69 | 0,59 |
| 1 | 101,08 | 6,41 | 0,85 | 1,00 | 2,76 |
| 1,5 | - | 14,75 | 4,70 | 1,38 | 7,57 |
| 2 | - | 23,92 | 10,01 | 5,26 | 13,01 |
| 3 | - | 42,07 | 22,65 | 20,61 | 23,64 |
| 4 | - | 57,78 | 35,87 | 33,37 | 32,80 |
| 5 | - | 70,11 | 48,18 | 44,82 | 41,13 |
| 6 | - | 79,16 | 58,83 | 55,28 | 48,49 |
| 7 | - | 85,44 | 67,55 | 64,15 | 54,84 |
| 8 | - | 89,62 | 74,38 | 71,03 | 60,66 |
| 9 | - | 92,29 | 79,53 | 76,16 | 65,48 |
| 10 | - | 93,94 | 83,29 | 79,88 | 69,73 |
| 11 | - | 94,93 | 85,96 | 82,50 | 72,95 |
| 12 | - | 95,51 | 87,80 | 84,58 | 75,78 |
| 13 | - | 95,83 | 89,03 | 86,22 | 78,33 |
| 14 | - | 96,01 | 89,84 | 87,42 | 79,89 |
| 15 | - | 96,11 | 90,36 | 88,44 | 81,81 |
| 16 | - | 96,16 | 90,69 | 89,33 | 83,05 |
| 17 | - | 96,19 | 90,89 | 90,04 | 85,78 |

**Tabelle 2: Freisetzung von Propiverinhydrochlorid/Propiverin in Prozent - Beispiele 7 - 13 (n.b. - nicht bestimmt)**

| Zeit [h] | Beispiel 7 Pellet) Propiverin·HCl, unretard. Zitronensäure-kerne | Beispiel 8 Pellet) Propiverin·HCl Zitronensäure 1:12 | Beispiel 9 (Pellet) Propiverin-HCl, Eudragit RS/RL/S 1,5: 1 : 2,5 | Beispiel 10 (Sphäroidtabl.) Propiverin·HCl, Zitronensäure | Beispiel 11 (Gelmatrixtabl.) Propiverin·HCl, Weinsäure | Beispiel 12 (Gelmatrixtabl.) Propiverin-HCl, Adipinsäure | Beispiel 13 (Gelmatrixtabl .) ohne Säurezusatz |
|---|---|---|---|---|---|---|---|
| 0,08 | n.b. | n.b. | n.b. | n.b. | n.b. | n.b. | n.b. |
| 0,17 | n.b. | n.b. | n.b. | n.b. | n.b. | n.b. | n.b. |
| 0,25 | n.b. | n.b. | n.b. | n.b. | n.b. | n.b. | n.b. |
| 0,5 | 0,30 | 0,00 | 0,52 | 0,35 | 2,69 | 0,16 | 4,64 |
| 1 | 0,56 | 0,00 | 1,47 | 1,80 | 6,17 | 5,12 | 9,55 |
| 1,5 | 5,58 | 3,41 | 10,48 | 3,26 | 11,08 | 10,66 | 15,63 |
| 2 | 17,42 | 8,80 | 28,60 | 6,41 | 14,54 | 14,61 | 20,38 |
| 3 | 37,14 | 35,46 | 48,91 | 16,54 | 20,96 | 21,75 | 27,99 |
| 4 | 55,53 | 59,52 | 67,24 | 26,82 | 26,92 | 28,23 | 34,34 |
| 5 | 63,46 | 78,80 | 78,84 | 38,45 | 32,43 | 34,29 | 39,52 |
| 6 | 68,17 | 83,27 | 83,36 | 49,61 | 37,57 | 39,91 | 44,19 |
| 7 | 71,00 | 87,54 | 84,00 | 59,24 | 42,30 | 45,03 | 48,25 |
| 8 | 73,81 | 90,13 | 84,82 | 65,53 | 46,75 | 49,95 | 52,14 |
| 9 | 75,90 | 91,38 | 85,56 | 71,55 | 50,94 | 54,47 | 55,71 |
| 10 | 77,48 | 91,72 | 86,22 | 75,12 | 54,80 | 58,74 | 58,70 |
| 11 | 77,81 | 92,01 | 86,90 | 77,83 | 58,33 | 62,73 | 61,81 |
| 12 | 78,26 | 92,42 | 87,72 | 80,67 | 61,61 | 66,14 | 64,65 |
| 13 | 78,65 | 92,76 | 88,41 | 82,98 | 64,75 | 69,42 | 67,34 |
| 14 | 79,00 | 93,10 | 89,19 | 84,82 | 67,61 | 72,58 | 69,71 |
| 15 | 79,46 | 93,51 | 89,75 | 85,36 | 70,36 | 75,44 | 72,06 |
| 16 | 79,81 | 93,94 | 89,93 | 86,16 | 72,91 | 79,99 | 74,14 |
| 17 | 80,20 | 94,32 | 90,26 | 86,40 | 75,33 | 80,49 | 76,74 |

**Beispiel 6**

**Gehaltsbestimmung von Propiverin/Propiverinhydrochlorid mittels HPLC, Zitronensäure mittels potentiometrischer Titration und von 2-Propanol mittels GC**

1. Zur quantitativen Bestimmung von Propiverin/Propiverinhydrochlorid in unterschiedlichen Darreichungsformen wird eine wirkstoffspezifische HPLC-Methode verwendet, die es erlaubt, Matrixbestandteile von Analyten abzutrennen.
Die angewendete Methode führt zu richtigen Ergebnissen, da sie bezüglich ihrer Selektivität für den Analyten, Linearität im vorgegebenen Arbeitsbereich, Richtigkeit und Präzision valide ist, was mit Hilfe der üblichen Experimente gezeigt werden konnte.
Zur quantitativen Bestimmung von Propiverin/Propiverinhydrochlorid in den wirkstoffhaltigen Darreichungsformen wird beispielsweise eine 15,0 mg Propiverinhydrochlorid entsprechende Menge der fein pulverisierten Darreichungsform in einen 100 ml-Meßkolben exakt eingewogen, mit 50 ml Methanol sowie einem Tropfen 0,1 M Salzsäure versetzt und im Ultraschallbad ca. 10 min behandelt. Anschließend werden 40 ml Wasser zugegeben und die Suspension erneut ca. 10 min im Ultraschallbad behandelt. Nach dem Abkühlen auf Raumtemperatur wird mit Wasser bis zur Eichmarke aufgefüllt.
Als Referenzlösung werden 60,0 mg Propiverinhydrochlorid in einen 100 ml-Meßkolben exakt eingewogen, mit 50 ml Methanol sowie einem Tropfen 0,1 M Salzsäure versetzt und im Ultraschallbad ca. 10 min behandelt. Anschließend werden 40 ml Wasser zugegeben und die Lösung erneut ca. 10 min im Ultraschallbad behandelt. Nach dem Abkühlen auf Raumtemperatur wird mit Wasser bis zur Eichmarke aufgefüllt.
Die Chromatographie wird mit einer handelsüblichen Anlage, bestehend aus Pumpe, Autosampler, Säulenofen und UV/VIS-Detektor, bei einer Flußrate von 1,0 ml/min, 40 °C Säulentemperatur und einer Detektionswellenlänge von 220 nm ausgeführt, wobei die Laufzeit 5 min und die Menge an eingespritzter Probe- bzw. Referenzlösung 20 $\mu$l beträgt. Als stationäre Phase wird ein Reversed Phase-Material (LiChrospher 60 Select B, 5 $\mu$m, 125 x 4 mm, Fa. Merck) und eine mobile Phase, bestehend aus 56 Volumenteilen eines 10 mM Kaliumdihydrogenphosphatpuffers, pH 1,0 (mit 85%iger Phosphorsäure) sowie 44 Volumenteilen Acetonitril verwendet.
Die Quantifizierung von Propiverin/Propiverinhydrochlorid in der Probe erfolgt als Doppelbestimmung gegen den entsprechenden Peak im Chromatogramm der Referenzlösung bei gleicher Wellenlänge. Das Ergebnis wird als Masseprozent in der Darreichungsform angegeben.
2. Die quantitative Bestimmung von Zitronensäure in den unterschiedlichen Darreichungsformen erfolgt mit Hilfe einer potentiometrischen Titration des ersten Äquivalenzpunktes der Zitronensäure.
Die angewandte Methode wurde bezüglich ihrer Spezifität, Richtigkeit und Präzision validert, wobei gezeigt werden konnte, dass Hilfsstoffe oder Propiverinhydrochlorid das Ergebnis nicht verfälschen.
Zur Durchführung wurden 50,0 mg der fein pulverisierten Darreichungsform exakt eingewogen, mit 50 ml demineralisiertem Wasser versetzt und ca. 5 min im Ultraschallbad behandelt. Anschließend wurde mit 0,1 M Natronlauge bis zum ersten Äquivalenzpunkt titriert. 1 ml verbrauchte Natronlauge entsprechen 6,403 mg Zitronensäure.
3. Die quantitative Bestimmung von 2-Propanol in den Pelletformulierungen wird mittels Gaschromatographie durchgeführt. Diese Methode führt zu richtigen Ergebnissen, da sie bezüglich ihrer Selektivität für den Analyten, der Linearität im vorgegebenen Arbeitsbereich, der Richtigkeit und der Präzision valide ist, was mit Hilfe der üblichen Experimente gezeigt werden konnte.
Zur Bestimmung von 2-Propanol in den Pelletformulierungen werden 100 mg der jeweiligen Form in verschiedene Zentrifugenröhrchen eingewogen und mit 1,0 ml Dimethylformamid versetzt. Anschließend wird die Suspension 2 Minuten im Ultraschallbad extrahiert, 3 Minuten bei 10.000 UPM zentrifugiert und der Überstand in Vials abdekantiert.
Als Referenzlösung werden 100 mg 2-Propanol in einem 100 ml Meßkolben eingewogen und mit Dimethylformamid zu 100 ml aufgefüllt. 2,5 ml dieser Lösung werden mit Dimethylformamid zu 50 ml aufgefüllt (500 ppm bezogen auf die Pelletmasse in der Probelösung).
Die Chromatographie wird mit einem handelsüblichen Gaschromatographen ausgeführt, der mit einer Sptitinjektionsvorrichtung, einem temperierbaren Säulenofen sowie einem Flammenionisationsdetektor ausgestattet ist und mit Helium als Trägergas betrieben wird.
Als stationäre Phase wird z. B. eine BTR-CW-Säule von 10 m Länge, einem Innendurchmesser von 0,53 mm und einer Filmdicke von 1,0 $\mu$m verwendet. Bei einem Säulendurchfluß von 6 ml/min, einem Einspritzvolumen von 1 $\mu$l und einer Säulentemperatur von 60 °C eluiert 2-Propanol nach ca. 0,6 min.
Die Quantifizierung von 2-Propanol in der Probe erfolgt als Doppelbestimmung gegen den entsprechenden Peak im Chromatogramm der Referenzlösung. Das Ergebnis wird als ppm in der Darreichungsform angegeben.

**Beispiel 7 (Vergleich)**

**Retardierung der Zitronensäuresphäroide**

In gleicher Art und Weise, wie bereits im Beispiel 1 beschrieben, werden bei gleicher Ansatzgröße 3990 g (entsprechend 96,7 % der Theorie, bezogen auf lösungsmittelfreies Material) sphärischer Zitronensäurekerne erhalten.

Zum Auftragen des Wirkstoffes werden 3650 g der unretardierten sphärischen Zitronensäurekerne bei einer Lufttemperatur von 45°C mittels 2-Stoffdüsen in der Wirbelschicht mit einer isopropanolisch-wäßrigen Suspension, bestehend aus 1200 g Propiverinhydrochlorid, 260 g Polyvinylpyrrolidon (Kollidon® K25), 45 g Zitronensäure, 185 g Talk, 45 g Magnesiumstearat, 1940 g 2-Propanol und 370 g demineralisiertem Wasser besprüht. Als Ausbeute wurden 5250 g an wirkstoffhaltigen, unretardierten Zitronensäurepellets erhalten (97,5 %, bezogen auf lösungsmittelfreies Material).

3500 g der so erhaltenen Wirkstoffpellets werden im nächsten Technologieschritt in der Wirbelschicht bei einer Zulufttemperatur von 40 - 45 °C mittels 2-Stöffdüsen mit einer isopropanolisch-wäßrigen Suspension von 720 g Eudragit® RS 12,5 (90 g Trockensubstanz Eudragit® RS), 365 g Eudragit® RL 12,5. (entsprechend 45 g Eudragit® RL Trockensubstanz), 720 g Eudragit® S 12,5 (90 g Eudragit® S Trockensubstanz), 24 g Triethylcitrat und 144 g mikrofeinem Talk in 1700 g 2-Propanol und 264 g Wasser besprüht. Die Auswaage an retardierten Wirkstoffpellets betrug 3700 g. Das entspricht einer theoretischen Ausbeute von 95,0 %, bezogen auf das trockene Material.

2800 g der so erhaltenen Pellets werden unter identischen technologischen Bedingungen mit einer Suspension von 781 g Eudragit® S 12,5 (entsprechend 98 g Eudragit® S Trockensubstanz), 100 g Triethylcitrat und 58,5 g mikrofeinem Talkum, in 760 g 2-Propanol und 90 g Wasser besprüht. Die Auswaage an Pellets betrug 2960 g: Das entspricht einer Ausbeute von 100 % der Theorie, bezogen auf lösungsmittelfreie Pellets.

Vor der Abfüllung werden 2900 g der so erhaltenen Pellets unter Zugabe von 15 g Talk gemischt und anschließend gesiebt. Die Korngrößenfraktion 0,7 mm bis 1,25 mm (2650 g, entsprechend 91 % der Theorie) weist einen nach Beispiel 5 bestimmten Gehalt von 19,0 % an Propiverinhydrochlorid auf. Der Gehalt an Zitronensäure wurde mit Hilfe der in Beispiel 6 beschriebenen Titration zu 53 % bestimmt.

Aus den experimentell bestimmten Gehalten ergibt sich ein molares Wirkstoff-Zitronensäure-Verhältnis von 1 : 5,8. Die Freisetzung von Propiverinhydrochlorid aus den oben beschriebenen Pellets wurde mit Hilfe der in Beispiel 5 dargestellten Methode ermittelt. Die Ergebnisse sind in der dort aufgeführten Tabelle wiedergegeben.

**Beispiel 8 (Vergleich)**

**Pelletformulierung mit Propiverinhydrochlorid - molares Wirkstoff-Zitronensäure-Verhältnis 1 : 12**

In gleicher Art und Weise, wie bereits in Beispiel 1 beschrieben, werden bei gleicher Ansatzgröße 4019 g (entsprechend 97,4 % der Theorie, bezogen auf die getrocknete Substanz) sphärische Zitronensäurekerne erhalten.

3750 g der so erhaltenen Kerne werden in gleicher Art und Weise, wie bereits in Beispiel 1 beschrieben, mit einer isopropanolisch-wäßrigen Suspension, bestehend aus 600 g Eudragit® S 12,5 (entsprechend 75 g Eudragit® S), 600 g Eudragit® L 12,5 (entsprechend 75 g Eudragit® L), 20 g Triethylcitrat, 100 g Talk, 1500 g 2-Propanol und 300 g Wasser, retardiert. Die Ausbeute betrug 4000 g, das entspricht 99,5 % der Theorie, bezogen auf lösungsmittelfreies Material.

3500 g der so erhaltenen retardierten Zitronensäuresphäroide werden mittels 2-Stoffdüsen in der Wirbelschicht bei 45 °C Zulufttemperatur mit einer isopropanolisch-wäßrigen Suspension von 575 g Propiverinhydrochlorid, 250 g Polyvinylpyrrolidon (Kollidon® K25), 45 g Citronensäure, 175 g Talk, 45 g Magnesiumstearat, 1860 g 2-Propanol und 350 g Wasser besprüht. Als Ausbeute wurden 4490 g Wirkstoffpellets (entsprechen 97,8 %, bezogen auf lösungsmittelfreies Material) erhalten.

3500 g der so erhaltenen Wirkstoffpellets werden unter identischen technologischen Bedingungen mit einer isopropanolisch-wäßrigen Suspension, bestehend aus 720 g Eudragit® RS 12,5 (entsprechend 90 g Eudragit® RS Trockensubstanz), 365 g Eudragit® RL 12,5 (entsprechend 45 g Eudragit® RL Trockensubstanz), 720 g Eudragit® S 12,5 (entsprechend 90 g Eudragit® S Trockensubstanz), 24 g Triethylcitrat und 144 g mikrofeinem Talk in 1700 g

2-Propanol und 264 g Wasser (demineralisiert) besprüht. Die Auswaage betrug 3894 g der retardierten Wirkstoffpellets. Das entspricht einer Ausbeute von 100 % der Theorie, bezogen auf die getrocknete Substanz.

3100 g der so erhaltenen retardierten Wirkstoffpellets werden anschließend in gleicher Art und Weise mit einer Suspension von 865 g Eudragit® S 12,5 (108 g Eudragit® S Trockensubstanz), 110 g Triethylcitrat, 65 g Talk in 840 g 2-Propanol und 100 g Wasser besprüht. Es wurden 3380 g der zweifach lackierten Wirkstoffpellets (99,9 % der Theorie, bezogen auf lösungsmittelfreies Material) erhalten.

Vor der Abfüllung in Hartgelatinekapseln werden 3000 g der so erhaltenen Pellets unter Zugabe von 15 g Talk gemischt und anschließend gesiebt. Die Komgrößenfraktion von 0,7 mm bis 1,25 mm (2750 g, entsprechend 91,7 % der Theorie) weist einen nach Beispiel 6 bestimmten Gehalt von 9,8 % an Propiverinhydrochlorid auf. Der Gehalt an Zitronensäure wurde mit Hilfe der in Beispiel 6 beschriebenen Titration zu 55,2 % bestimmt. Daraus ergibt sich ein molares Wirkstoff-Zitronensäure-Verhältnis von 1 : 11,8.

Die Freisetzung von Propiverinhydrochlorid aus den oben beschriebenen Pellets wurde mit Hilfe der in Beispiel 5 dargestellten Methode bestimmt. Die Ergebnisse sind in der aufgeführten Tabelle dargestellt und zeigen, dass die Wirkstofffreisetzung ähnlich wie im Beispiel 3.1 in der ersten und zweiten Stunde stark reduziert ist. Im weiteren Verlauf macht sich jedoch die erhöhte Menge an Zitronensäure bemerkbar, die zu einem höheren osmotischen Druck und damit zu einer zu schnellen Wirkstofffreisetzung führt.

**Beispiel 9 (Vergleich)**

**Pelletformulierung mit Propiverinhydrochlorid - Eudragit-Verhältnis RS/RL/S 1,5:1:2,5**

In gleicher Art und Weise, wie bereits in Beispiel 1 beschrieben, werden bei gleicher Ansatzgröße 4000 g (entsprechend 97,0 % der Theorie, bezogen auf lösungsmittelfreies Material) sphärische Zitronensäurekerne erhalten.

Zur Retardierung werden 3750 g der so erhaltenen Starterkerne in gleicher Art und Weise mit einer Suspension der gleichen quantitativen Zusammensetzung, wie bereits in Beispiel 1 beschrieben, besprüht. Die Auswaage an retardierten Zitronensäurekernen betrug 3980 g, das entspricht einer Ausbeute von 99,0 %, bezogen auf lösungsmittelfreies Material.

3500 g der so erhaltenen retardierten Zitionensäuresphäroide werden mittels 2-Stoffdüsen in der Wirbelschicht bei 45 °C Zulufttemperatur mit einer isopropanolisch-wäßrigen Suspension von 1100 g Propiverinhydrochlorid, 250 g Polyvinylpyrrolidon (Kollidon® K25), 45 g Zitronensäure, 175 g Talk, 45 g Magnesiumstearat, 1860 g 2-Propanol und 350 g Wasser besprüht. Als Ausbeute wurden 4990 g Wirkstoffpellets (entsprechen 97,5 %, bezogen auf lösungsmittelfreies Material) erhalten.

4000 g der so erhaltenen Wirkstoffpellets werden unter identischen technologischen Bedingungen mit einer isopropanolisch-wäßrigen Suspension, bestehend aus 615 g Eudragit® RS 12,5 (entsprechend 77 g Eudragit® RS Trockensubstanz), 410 g Eudragit® RL 12,5 (entsprechend 51 g Eudragit® RL Trockensubstanz), 1025 g Eudragit® S 12,5 (entsprechend 128 g Eudragit® S Trockensubstanz), 27 g Triethylcitrat und 165 g mikrofeinem Talk in 1950 g 2-Propanol und 300 g Wasser (demineralisiert) besprüht. Die Auswaage betrug 4440 g der retardierten Wirkstoffpellets. Das entspricht einer Ausbeute von 99,8 % der Theorie, bezogen auf die getrocknete Substanz.

3100 g der so erhaltenen retardierten Wirkstoffpellets werden in gleicher Art und Weise und mit der gleichen Suspension, wie bereits in Beispiel 8 beschrieben, besprüht. Auf die Weise wurden 3100 g der lackierten Wirkstoffpellets (91,6 % Ausbeute der Theorie, bezogen auf lösungsmittelfreies Material) erhalten.

Vor der Abfüllung in Hartgelatinekapseln werden 2500 g der so erhaltenen lackierten Wirkstoffpellets unter Zugabe von 15 g mikrofeinem Talk gesiebt. Die Korngrößenfraktion von 0,7 mm bis 1,25 mm (2450 g, entsprechend 98,0 % der Theorie) weist einen nach Beispiel 6 bestimmten Gehalt von 18,5 % an Propiverinhydrochlorid auf. Der Gehalt an Zitronensäure wurde mit Hilfe der in Beispiel 6 beschriebenen Titration zu 49 % bestimmt. Somit ergibt sich hiermit das im üblichen Rahmen liegende molekulare Verhältnis von Propiverin zu Zitronensäure von 1 : 5,6.

Die Freisetzung von Propiverinhydrochlorid aus den oben beschriebenen lackierten Pellets wurde mittels der im Beispiel 5 beschriebenen Methode bestimmt. Die Ergebnisse der Freisetzung sind ebenfalls dort wiedergegeben.

**Beispiel 10**

**Sphäroidtablettenformulierung mit Propiverinhydrocblorid**

Zur Herstellung einer Tablette aus magensaftresistenten Sphäroidteilchen wurden in einem Rhönradmischer 1,25 kg Propiverinhydrochlorid (Korngröße kleiner als 0,25 mm), 2,97 kg Citronensäure (Korngröße kleiner als 0,25 mm), 0,80 kg Polyvinylpyrrolidon (Kollidon® K 25), 1,44 kg Lactose (Tablettose®) 5 min gemischt. Zur Zerteilung von Agglomeraten wurde die Mischung über ein Lochsieb mit einem Lochdurchmesser von 0,81 mm gegeben und nochmals 5 min gemischt. Anschließend wurde zu dieser Mischung über ein Sieb mit der Maschenweite von 0,5 mm 0,05 kg Magnesiumstearat zugesetzt und nochmals 2 min die gesamte Masse gemischt. Die Mischung hat ein molares Verhältnis von Propiverinhydrochlorid zu Zitronensäure von 1:5.

Die so erhaltene Mischung wurde kompaktiert und anschließend die erhaltenen Teile gebrochen. Die Fraktion 0,6-1,2mm wurde abgesiebt. Der entstandene Feinanteil wurde wiederholt kompaktiert, gebrochen und gesiebt, bis die gesamte Menge in Granulatteilchen der angegebenen Größe vorlag. Die Ausbeute betrug 5,28 kg, entsprechend 81,1 % der Theorie. 3,5 kg der erhaltenen Granulatteilchen wurden in der Wirbelschicht mit einer wäßrigen Suspension von 967 g Eudragit® NE 30 D (entsprechend 290 g Trockenmasse), 467 g Eudragit® L 30D (entsprechend 140 g Trockenmasse), 100 g Talkum und 3300 g Wasser mit einer 2-Stoffdüse bei einer Zulufttemperatur von 50°C besprüht und anschließend bei einer Zulufttemperatur von 40°C und verringerter Luftmenge nachgetrocknet. Die Ausbeute betrug 3,985 kg, entsprechend 93,8 % der Theorie, bezogen auf lösungsmittelfreies Material.

3,0 kg der so erhaltenen retardierten Teilchen wurden in einem Rhönradmischer mit 5,0 kg mikrokristalliner Cellulose (Typ 101); 0,52 kg Polyvinylpyrrolidon (Kollidon® K 25) und 1,0 kg Crospovidon XL 20 min gemischt. Anschließend wurde der Mischung 0,1 kg Magnesiumstearat über ein Sieb mit der Maschenweite 0,50 mm zugesetzt und nochmals 5 min gemischt.

Die so erhaltene Preßmischung wurde auf einer Rundläuferpresse mit einem oblongen Stempelwerkzeug (Länge 19 mm, Breite 8,5 mm, Radius der Wölbung 8 mm) zu Tabletten mit einer Masse 865 mg und einer Bruchfestigkeit von 100-140 N verpreßt.

Mittels der im Beispiel 6 beschriebenen Methoden wurde ein Gehalt am Propiverinhydrochlorid von 5,2 % und an Zitronensäure von 11,8 % bestimmt. Daraus ergibt sich ein molares Verhältnis von Wirkstoff zu Zitronensäure von 1 : 4,8. Auch dieser Unterschied ist durch Abrieb- und Sprühverluste innerhalb der verschiedenen Technologieschritte zu erklären.

Die Freisetzung von Propiverinhydrochlorid aus den oben beschriebenen Tabletten wurde mit Hilfe der im Beispiel 5 beschriebenen Methode bestimmt. Die gefundenen Werte sind in der dort angegebenen Tabelle dokumentiert.

**Beispiel 11**

**Gelmatrixtablettenformulierung mit Propiverinhydrochlorid und Weinsäure**

Zur Herstellung einer magensaftresistenten Gelmatrixtablette mit Propiverinhydrochlorid und Weinsäure werden 132,5 g Weinsäure mit einer Korngröße von 100 % kleiner als 250 μm, 112,5 g Propiverinhydrochlorid, 187,5 g Hypromellose (Methocel® K100) und 62,5 g microkristalline Cellulose 10 min im Rhönradmischer gemischt, über ein Lochsieb mit einem Lochdurchmesser von 0,81 mm gegeben und erneut 10 min gemischt. Ein Teil der Vermischung wird mit 5,0 g Magnesiumstearat angerieben und die resultierende Verreibung über ein Sieb mit 500 μm Maschenweite zur restlichen Vormischung gegeben. Anschließend wird erneut 2 min gemischt.

Die so erhaltene Preßmischung wird auf einer Rundläuferpresse mit 8 mm-Werkzeug (Radius der Wölbung 9 mm) zu Tablettenkernen mit einer Bruchfestigkeit von 50 N - 70 N und einem Abrieb von kleiner als 0,5 % verpreßt.

350 g der so erhaltenen Tablettenkerne werden in der Wirbelschicht mit Hilfe von 2-Stoffdüsen mit einer Suspension von 48 g Eudragit® L 12,5 (6,0 g Eudragit® L Trockensubstanz), 60 mg Magnesiumstearat, 600 mg Talkum und 600 mg Triethylcitrat in 40 g 2-Propanol besprüht.

Es wurden 355 g der magensaftresistenten Gelmatrixtabletten erhalten, was einer Ausbeute von 99,4 % entspricht.

Der Gehalt an Propiverinhydrochlorid wurde mit Hilfe der in Beispiel 6 beschriebenen Methode zu 22,3 % bestimmt. Daraus ergibt sich ein molares Wirkstoff-Weinsäure-Verhältnis von 1 : 3,2. Die Freisetzung von Propiverinhydrochlorid aus den oben beschriebenen Matrixtabletten wurde mit Hilfe der in Beispiel 5 beschriebenen Methode bestimmt und ist in der dortigen Tabelle wiedergegeben.

**Beispiel 12**

**Gelmatrixtablettenformulierung mit Propiverinhydrochlorid und Adipinsäure**

Zur Herstellung einer magensaftresistenten Gelmatrixtablette mit Propiverinhydrochlorid und Adipinsäure werden 132,5 g Adipinsäure mit einer Korngröße von 100 % kleiner als 250 μm, 112,5 g Propiverinhydrochlorid, 187,5 g Hypromellose (Methocel® K100) und 62,5 g microkristalline Cellulose 10 min im Rhönradmischer gemischt, über ein Lochsieb mit einem Lochdurchmesser von 0,81 mm gegeben und erneut 10 min gemischt. Ein Teil der Vermischung wird mit 5,0 g Magnesiumstearat angerieben und die resultierende Verreibung über ein Sieb mit 500 μm Maschenweite zur restlichen Vormischung gegeben Anschließend wird erneut 2 min gemischt.

Die so erhaltene Preßmischung wird auf einer Rundläuferpresse mit 8 mm-Werkzeug (Radius der Wölbung 9 mm) zu Tablettenkernen mit einer Bruchfestigkeit von 50 N - 70 N und einem Abrieb von kleiner als 0,5 % verpreßt.

350 g der so erhaltenen Tablettenkerne werden in der Wirbelschicht mit Hilfe von 2-Stoffdüsen mit einer Suspension von 48 g Eudragit® L 12,5 (6,0 g Eudragit® L Trockensubstanz), 60 mg Magnesiumstearat, 600 mg Talkum und 600 mg Triethylcitrat in 40 g 2-Propanol besprüht.

Es wurden 356 g der magensaftresistenten Gelmatrixtabletten erhalten, was einer Ausbeute von 99,6 % entspricht.

Der Gehalt an Propiverinhydrochlorid wurde mit Hilfe der in Beispiel 6 beschriebenen Methode zu 22,7 % bestimmt. Das molare Wirkstoff-Adipinsäure-Verhältnis beträgt ebenfalls 1 : 3,2. Die Freisetzung von Propiverinhydrochlorid aus den oben beschriebenen Matrixtabletten wurde mit Hilfe der im Beispiel 5 beschriebenen Methode bestimmt und ist in der dortigen Tabelle wiedergegeben.

**Beispiel 13**

**Gelmatrixtablettenformulierung mit Propiverinhydrochlorid ohne Säurezusatz**

Zur Herstellung einer modifizierten Gelmatrixtablette ohne saure Substanzen mit Propiverinhydrochlorid werden 45 g Propiverinhydrochlorid mit einer Korngröße kleiner 0,25 mm, 247 g microkristalliner Cellulose (Typ 101) und 67 g Hypromellose (Methocel® K 100) im Rhönradmischer 10 min gemischt, über ein Lochsieb mit einem Lochdurchmesser von 0,25 mm gegeben und erneut 10 min gemischt. Ein Teil der Vermischung wird mit 3,6 g Magnesiumstearat angerieben und die resultierende Verreibung über ein Sieb mit der Maschenweite von 0,25 mm zur restlichen Vormischung gegeben. Anschließend wird erneut 2 min im Rhöhnradmischer gemischt.

Die so erhaltene Mischung wird auf einer Rundlauftablettenpresse mit 8 mm-bikonvexen Werkzeug (Radius der Wölbung 9 mm) und einer Bruchkerbe im Oberstempel zu Tablettenkernen mit einer Bruchfestigkeit von 100 - 150 N, einer mittleren Masse von 244 mg und einem Abrieb kleiner 0,5 % verpreßt.

300 g der so erhaltenen Tablettenkerne werden in der Wirbelschicht mit Hilfe von 2-Stoffdüsen mit einer Suspension von 40 g Eudragit® L 12,5 (entsprechend 5,0 g Eudragit® L Trockensubstanz), 0,05 g Magnesiumstearat, 0,5 g Talkum und 0,5 g Triethylcitrat in 60 g 2-Propanol besprüht.

Es wurden 304 g der besprühten Gelmatrixtabletten erhalten, was einer Ausbeute von 99 % entspricht.

Der Gehalt an Propiverinhydrochlorid wurde mit Hilfe der im Beispiel 6 beschriebenen Methode zu 12,2 % bestimmt. Daraus ergibt sich ein Gehalt von 30,3 mg Propiverinhydrochlorid pro Tablette. Deshalb wurden als Referenzsubstanz in der Freisetzungsbestimmung, durchgeführt nach Ausführungsbeispiel 5, anstatt 45 mg nur 30 mg Propiverinhydrochlorid verwendet. Die Gehaltskorrektur wurde bei der Freisetzungsberechnung berücksichtigt. Alle anderen Freisetzungsparameter blieben unverändert. Die gefundenen Freisetzungswerte sind in der Tabelle des Beispiels 5 als Mittelwerte einer Sechsfachbestimmung enthalten.

**Beispiel 14**

**Vergleichende Bioverfügbarkeitsstudie der Pelletformulierungen der Beispiele 1, 2 und 3.1**

In einer klinischen Studie wurden die Bioverfügbarkeit und die Pharmakokinetik der Pelletformulierungen der Beispiele 1, 2 und 3.1 verglichen.

Dazu erhielten 6 Probanden die Pelletformulierungen ä 45 mg Propiverinhydrochlorid im Cross-over-Design jeweils als Einzelgabe. Die Blutspiegel wurden zu 25 Zeitpunkten in Abständen von 20 min - 12 Stunden über insgesamt 48 Stunden verfolgt. Propiverin und dessen Hauptmetabolit Propiverin-N-Oxid wurden mittels einer validierten HPLC-Methode im Serum bestimmt. Hierzu wurden 0,5 ml der tiefgefrorenen Serum- bzw. Kontrollproben nach dem Auftauen mit 0,5 ml Phosphorsäure (4 %) versetzt und danach mittels Festphasen (Nexus cartridges, 1 ml, 30 mg) extrahiert. Die Eluate wurden zur Trockne eingedampft und in 100 μl mobiler Phase aufgenommen.

Die Chromatographie wurde mit einer handelsüblichen Anlage, bestehend aus Pumpe, Autosampler, Säulenofen und Diodenarray-Detektor bei einer Flußrate von 1,2 ml/min, einer Säulentemperatur von 40 °C und einer Detektionswellenlänge von 202 nm durchgeführt, wobei die Laufzeit ca. 5 Minuten und die Menge an Proben- bzw. Referenzlösung (gleichbehandelte Leerserumprobe unter Zusatz von Propiverinhydrochlorid und Propiverin-N-Oxid) 20 μl betrug. Als stationäre Phase wurde ein Reversed-Phase-Material (Vorsäule: LiChrocart 10 x 2 mm, LiChrospher 60, RP-select B, 5 μm (Merck); Trennsäule: LiChrospher 60 - 5, select B, 125 x 2mm (Macherey-Nagel)) und als mobile Phase ein Gemisch von 70 Volumenanteilen Acetonitril und 30 Volumentanteilen Phosphatpuffer pH 7,3 (2 mM Kaliumdihydrogenphosphat und Dinatriumhydrogenphosphat) verwendet. Die Datenerfassung und - auswertung erfolgte mittels Chromeleon Chromatography Data System.

Unter diesen Analytik-Bedingungen betrug die Wiederfindung für Propiverin 99 % und für das Propiverin-N-Oxid 95 %. Bei wiederholter Messung (n = 5) von gespikten Serumproben (10 ng/ml Propiverin bzw. 20 ng/ml N-Oxid) betrug der Variationskoeffizient der gemessenen Konzentrationen einheitlich 6 %.

Mit den gemessenen Konzentrationen wurden Konzentrations-Zeit-Kurven (Blutspiegel) über einen Zeitraum von 48 Stunden erstellt und die Fläche unter diesen Kurven berechnet (Area Under the Curve = AUC). Dieser Parameter ist ein Maß für die über die Zeit im Blutkreislauf verfügbare Menge an Propiverin bzw. Propiverin-N-oxid (Bioverfügbarkeit).

Die mittels dieser Bioverfügbarkeitsstudie erhaltenen AUC-Werte für Propiverin (siehe Tabelle) belegen, dass eine Retardierung der Arzneistoff-Freisetzung (Beispiele 2 und 3.1) nicht zu einer Verminderung der Bioverfügbarkeit im Vergleich zu einer sofort-freisetzenden Pelletformulierung (Beispiel 1) führt. Somit ist gezeigt, dass bei Gabe von Propiverin in Form der erfindungsgemäßen Retardformulierungen die Verfügbarkeit von Propiverin auch aus tieferen Darmabschnitten erhalten bleibt. Das heißt, bei Freisetzung des Propiverins in tieferen Darmabschnitten tritt die überlicherweise für basische Arzneistoffe zu beobachtende und aufgrund der bekannten physikochemischen Eigenschaften von Propiverin zu erwartende Verminderung der Bioverfügbarkeit nicht auf.

Entgegen der Vermutung, dass dafür eine in tiefen Darmabschnitten unveränderte Propiverin-Resorption ursächlich sein könnte, wurde als Ursache überraschend die verminderte Umwandlung zum Propiverin-N-Oxid (Metabolit) gefunden. Die Menge (AUC) des gebildeten Propiverin-N-Oxids bzw. das Verhältnis Metabolit/Muttersubstanz nimmt mit zunehmender Retardierung ab (siehe Tabelle). Somit kommt es bei Gabe von retardierten oralen Darreichungsformen von Propiverin oder seiner pharmazeutisch annehmbarer Salze bei gleicher Bioverfügbarkeit der Wirksubstanz Propiverin zu einer verminderten systemischen Belastung des Organismus mit dem unerwünschten Metabolisierungsprodukt Propiverin-N-Oxid.

Als klinisch vorteilhaft für eine individuell sichere Dosierung ist die überraschend gefundene Tatsache anzusehen, dass die interindividuelle Variabilität der verfügbaren Propiverinmenge (AUC), ausgedrückt als Variationskoeffizient (VK), mit der Retardierung sehr deutlich abnimmt (siehe Tabelle). Von 62 % für die sofort-freisetzende Pelletformulierung des Beispiels 1 erfolgt eine Verminderung auf 27 % für die am stärksten retardierte Formulierung des Beispiels 3.1.

In den Abbildungen 1 und 2 sind die Konzentrations-Zeit-Verläufe (Blutspiegel) von Propiverin bzw. Propiverin-N-Oxid nach Gabe der Pelletformulierungen der Beispiele 1, 2 und 3.1 als Mittelwertskurven der 6 untersuchten Probanden dargestellt. Zum Vergleich ist der Blutspiegel nach Gabe von 3 Dragees (ä 15 mg Propiverinhydrochlorid) des Handelspräparates Mictonorm® (Mittelwertskurve von 34 Probanden) ebenfalls dargestellt. Danach kann die Pelletformulierung des Beispiels 1 als Referenz für das Handelspräparat gelten.

Die Blutspiegel der Abbildungen 1 und 2 zeigen, dass die Retardierung zu einer drastischen Abnahme der Geschwindigkeit des Konzentrationsanstieges von Propiverin und Propiverin-N-Oxid führt. Zusätzlich ist die Höhe des Konzentrationsmaximums vermindert (Beispiel 1 versus Beispiel 2). Bei starker Retardierung, wie der des Beispiels 3.1, ist ein diskretes Konzentrationsmaximum vorteilhafterweise nicht mehr zu beobachten, d. h., es entsteht ein abgeflachter Blutspiegel mit relativ konstanten Konzentrationen über einen langen Zeitraum, ohne dass die Bioverfügbarkeit durch die Retardierung vermindert wird.

Im weiteren ist ersichtlich, dass durch die erfindungsgemäßen Darreichungsformen, beispielsweise die des Beispiels 3.1 sowie unter Beachtung der in-vitro/in-vivo-Korrelation, klinisch wirksame Blutspiegel über 24 Stunden realisiert werden können.

Zusätzlich ist durch das Vermeiden von Konzentrationsspitzen eine Verringerung der Häufigkeit und/oder Schwere von anticholinergen Nebenwirkungen zu erwarten.

Die Ergebnisse zur Bioverfügbarkeitsstudie nach Gabe der Pelletformulierungen der Beispiele 1, 2 und 3.1 sind als Mittelwerte in folgender Tabelle zusammengestellt.

**Tabelle: Bioverfügbarkeit (AUC) von Propiverin und Propiverin-N-oxid**

| Parameter | Pellet-formulierung Beispiel 1 | Pellet-formulierung Beispiel 2 | Pellet-formulierung Beispiel 3.1 |
|---|---|---|---|
| Propiverin $AUC_{0-48h}$ [$ng \cdot h \cdot ml^{-1}$] VK [%] | 1667 62 | 1705 47 | 1596 27 |
| Propiverin-N-Oxid $AUC_{0-48h}$ [$ng \cdot h \cdot ml^{-1}$] | 13076 | 8779 | 7829 |
| AUC-Verhältnis Propiverin-N-Oxid/ Propiverin | 7,8 : 1 | 5,15 : 1 | 4,9 : 1 |
| AUC von Propiverin-N-Oxid bezogen auf Beispiel 1 [%] | 100 | 71 | 63 |

**Beispiel 15**

**Bioäquivalenzstudie der Pelletformulierung des Beispiels 3.2 im Vergleich zum Handelspräparat Mictonorm®**

In einer zulassungsrelevanten, Guideline-gerechten Bioäquivalenzstudie wurde die Bioverfügbarkeit von Propiverin aus der schnell-freisetzenden Handelsform (Mictonorm®) mit der der Pelletformulierung des Beispiels 3.2 verglichen.

Dazu erhielten 12 männliche und 12 weibliche gesunde Probanden randomisiert im Cross-over-Design über 7 Tage entweder 3mal täglich ein Dragee Mictonorm® (à 15 mg Propiverinhydrochlorid) oder 1 mal täglich die Pelletformulierung des Beispiels 3.2 (45 mg Propiverinhydrochlorid). Der Wechsel der Medikation erfolgte nach einer Auswaschphase von 14 Tagen. Am jeweils siebten Tag wurden die Steady-state-Blutspiegel zu 28 Zeitpunkten in Abständen von 30 min - 2 Stunden über insgesamt 24 Stunden verfolgt. Propiverin und dessen Hauptmetabolit Propiverin-N-Oxid wurden mittels der in Beispiel 13 beschriebenen HPLC-Methode im Serum bestimmt.

Mit den gemessenen Konzentrationen wurden Konzentrations-Zeit-Kurven unter den Bedingungen der wiederholten Gabe (Steady-state-Blutspiegel) über einen Zeitraum von 24 Stunden erstellt und die Fläche unter diesen Kurven berechnet (Area Under the Curve = $AUC_{0-24\ h,ss}$). Dieser Parameter ist ein Maß für die über 24 Stunden im Blutkreislauf verfügbare Menge an Propiverin bzw. Propiverin-N-oxid.

Die Ergebnisse (siehe Tabelle Daten zur Bioäquivalenz) bestätigen auch für Steady-state-Bedingungen die bereits in Beispiel 13 gemachte Beobachtung, wonach die Bioverfügbarkeit von Propiverin unverändert bleibt, wenn es in Form von retardierten Pelletformulierungen verabreicht wird. Es besteht Bioäquivalenz zwischen dem Handelspräparat (3 x 15 mg) und der Pelletformulierung des Beispiels 3.2 (1x 45 mg). Auch die über 24 Stunden gemittelten Serumkonzentrationen sind gleich (siehe $C_{average}$ in der Tabelle).

Darüberhinaus bestätigt sich der aus Beispiel 13 erwartete Vorteil einer verminderten interindividuellen Variabilität der Bioverfügbarkeit von Propiverin bei Gabe der Pelletformulierung im Vergleich zum Handelspräparat. Der Variationskoeffizient für die Propiverin-AUC beträgt für die Pelletformulierung lediglich 15 % (Handelspräparat: 31 %). Damit ist klinisch eine individualisierte Dosierung möglich.

Bei allen 24 Probanden kommt es im intraindividuellen Vergleich zu einer Abnahme der AUC des Propiverin-N-Oxides bei Gabe der Pelletformulierung im Vergleich zum Handelspräparat. Damit ergibt sich ein signifikant kleinerer Mittelwert der AUC nach Gabe der Pelletformulierung im Vergleich zu der des Handelspräparates. Auch die über 24 Stunden gemittelte Serumkonzentration ist bei Gabe der Pelletformulierung signifikant niedriger (siehe $C_{average}$ in der Tabelle). Damit ist die in Beispiel 13 beschriebene Verminderung der Belastung mit dem klinisch nicht erforderlichen Umwandlungsprodukt Propiverin-N-Oxid für die Bedingungen der wiederholten Gabe (steady state) bestätigt.

Die unveränderten Propiverin-Werte für AUC und $C_{average}$ bei Gabe der Pelletformulierung beweisen auch, dass es durch die retardierte Freisetzung nicht zu einer Akkumulation von Propiverin im Sinne eines Blutspiegelanstiegs über die Zeit kommt.

**Tabelle: Daten zur Bioäquivalenz**

| Parameter | Mictonorm® 3x15 mg | Pelletformulierung Beispiel 3.2 1 × 45 mg |
|---|---|---|
| Propiverin $AUC_{0-24\ h,\ ss}$ [$ng\text{-}h\text{-}ml^{-1}$] VK [%] | 1677 31 | 1711 15 |
| Propiverin-N-Oxid $AUC_{0-24\ h,\ ss}$ [$ng\text{-}h\text{-}ml^{-1}$] | 11080 | 9316* |
| AUC-Verhältnis Propiverin-N-Oxid/ Propiverin | 6.6 : 1 | 5.4 : 1 |
| $AUC_{0-24\ h,\ ss}$ von Propiverin-N-Oxid bezogen auf Mictonorm® [%] | 100 | 84* |
| Propiverin $C_{average}$ [ng/ml] | 69.8 | 71.3 |
| Propiverin-N-Oxid $C_{average}$ [ng/ml] | 462 | 388* |
| * signifikant kleinerer Wert als bei Gabe des Handelspräparates Mictonorm | | |

Neben den Daten zur Bioverfügbarkeit und Pharmakokinetik wurden in dieser Studie auch die Nebenwirkungen erfaßt. In der nachfolgenden Tabelle ist die Häufigkeit der Nebenwirkungen aufgeführt, für die ein Zusammenhang mit der Gabe von Propiverin durch einen Arzt als "sicher", "wahrscheinlich" oder zumindest als "möglich" eingestuft wurde. Unter der Pelletformulierung ist die Häufigkeit von für Propiverin typischen anticholinergen Nebenwirkungen um fast die Hälfte (Akkomodationsstörungen und gesteigerte Lichtempfindlichkeit) bzw. um ein Viertel (Mundtrockenheit) vermindert. Die Gesamthäufigkeit aller berichteten Nebenwirkungen ist um etwa ein Drittel vermindert.

**Tabelle: Nebenwirkungen**

| Art der Nebenwirkung (NW) | Mictonorm® 3×15mg | Pelletformulierung Beispiel 3.2 1 x 45 mg |
|---|---|---|
| für Propiverin typische anticholinerge NW: | | |

(fortgesetzt)

| Art der Nebenwirkung (NW) | Mictonorm® 3×15mg | Pelletformulierung Beispiel 3.2 1 x 45 mg |
|---|---|---|
| a) Akkomodationsstörungen/ge-steigerte Lichtempfindlichkeit<br>b) Mundtrockenheit | 19<br>20 | 10 (53 %)<br>15 (75 %) |
| Summe anderer NW | 12 | 8 (66 %) |
| Summe aller NW | 51 | 33 (65 %) |

**Beispiel 16**

**In vitro-/in vivo-Korrelation**

Zur Simulation der Freisetzungsvorgänge von Wirkstoffen aus verschiedenen Darreichungsformen wird das in vitro-Freisetzungsverhalten der Darreichungsformen mit den in vivo-Daten korreliert. Kann eine Korrelation von den in vivo- und in vitro-Daten gezeigt werden, ist eine Vorhersage des in vivo-Freisetzungsverhaltens anderer Darreichungsformen aufgrund ihres in vitro-Freisetzungsverhaltens möglich.

Als erste Voraussetzung für eine in vivo-/in vitro-Korrelation muß gezeigt werden, dass der in vitro-Freisetzungsmechanismus für die betrachteten Arzneiformen identisch ist. Dies wird durch die Homomorphie (Formgleichheit) der entsprechenden Freisetzungsprofile gezeigt.

Dazu wurden die nach Beispiel 5 bestimmten in vitro-Freisetzungsdaten der in Beispiel 2 und Beispiel 3.1 beschriebenen Propiverinhydrochloridhaltigen Pelletformulierungen mit Hilfe einer Weibull-Funktion beschrieben:

$$M_{(t)} = M_0(1 - e^{-\{(\lambda \bullet (t-\tau))\}^{\beta}})$$

*M(t)* = Menge an freigesetztem Propiverinhydrochlorid zum Zeitpunkt t
mit $M_0$ = Gesamtfreisetzungsmenge Propiverinhydrochlorid [%]
λ = Freisetzungskonstante [1/h]
β = Steigerungsfaktor
τ = Verschiebungsfaktor der Funktion auf der Zeitachse (lag-time) [h]

Die Anpassung der Kurven erfolgte für alle Darreichungsformen separat und wurde mit Hilfe einer geeigneten Software, beispielsweise HOEGIP-PC-Software, nach der Methode der kleinsten Fehlerquadrate durchgeführt. Für den mathematischen Vergleich der beiden in vitro-Freisetzungsprofile wurden die Kurven auf 100 % freigesetztes Propiverinhydrochlorid bei Abbruch der Experimente normiert. Anschließend wurden die Zeitwerte mit Hilfe der folgenden linearen Transformation angepasst:

$$t_{i,Bsp2,trans} = (t_{i,Bsp2} - \tau_{Bsp2}) \cdot (\lambda_{Bsp2} / \lambda_{Bsp3.1}) + \tau_{Bsp3.1}$$

bzw.

$$t_{i,Bsp3.1,trans} = (t_{i,Bsp3.1} - \tau_{Bsp3.1}) \cdot (\lambda_{Bsp3.1} / \lambda_{Bsp2}) + \tau_{Bsp2}$$

mit

| | |
|---|---|
| $t_{i,Bsp2,trans}$ = | Transformierter Zeitweit, des i-ten Meßwertes zur Zeit $t_i$ der Darreichungsform nach Beispiel 2 |
| $t_{i,Bsp2}$ = | Nicht transformierter Zeitpunkt des i-ten Meßwertes der in vivo-Freisetzung des Beispiels 2 |

$\tau_{Bsp2}$ = Lag-Time der Freisetzung des Beispiels 2

$\lambda_{P2}$ = Freisetzungskontraste für die Meßwerte der Darreichungsform nach Beispiel 2

Analoges gilt für die Indices des Beispiels 3.1.

Die Ergebnisse sind in den Abbildungen 3 (Transformation von Beispiel 2 auf Beispiel 3.1) und 4 (Transformation von Beispiel 3.1 auf Beispiel 2) dargestellt. Die Korrelationskoeffizienten für die Transformation betrugen 0,9997 für die Abbildung von Beispiel 2 auf Beispiel 3.1 bzw. 0,99969 für die Transformation von Beispiel 3.1 auf Beispiel 2. Dieser Wert zeigt die nahezu exakte Formgleichheit der betrachteten in vitro-Freisetzungsprofile. Die Voraussetzung für einen Vergleich der in vivo-Daten mit den in vitro-Daten ist damit gegeben.

In einem nächsten Schritt werden die bereits in Beispiel 13 dargestellten mittleren Serumspiegel von Propiverin aus 6 Probanden nach Einmalgabe der Darreichungsformen entsprechend Beispiel 2 bzw. Beispiel 3.1 mit Hilfe der Methode der Dekonvolution zu einem kumulativen in vivo-Freisetzungsprofil addiert. Dazu werden die jeweils pro Zeiteinheit im Serum verfügbaren Wirkstoffmengen unter Berücksichtigung des metabolischen Abbaus des Wirkstoffes über die Zeit betrachtet.

Anschließend wurde versucht, die so erhaltenen in vitro-Freisetzungsprofile mit Hilfe der bereits oben beschriebenen linearen Transformation der Zeitachse auf die in vivo-Freisetzungsprofile abzubilden.

Für die in vivo- bzw. in vitro-Freisetzungsprofile der Darreichungsformen entsprechend Beispiel 2 und 3.1 sind die Ergebnisse dieser Verfahrensweise in den Abbildungen 5 und 6 dargestellt. Es ist zu erkennen, dass die dargestellten Kurven bei beiden betrachteten Darreichungsformen eine gute Übereinstimmung der Freisetzungsprofile aufweisen.

Somit konnte gezeigt werden, dass die in vivo-Freisetzungsprofile der Beispiele 2 und 3.1 mit Hilfe von geeigneten in vitro-Freisetzungsexperimenten bestimmt werden können. Damit lassen sich aus den erhaltenen in vitro-Freisetzungsprofilen Rückschlüsse auf das in vivo-Freisetzungsverhältnis von nicht am Menschen getesteten Darreichungsformen generieren und deren Brauchbarkeit zur Erzeugung klinisch relevanter Blutspiegel und somit ihrer Praxisrelevanz vorhersagen.

## Patentansprüche

1. Pharmazeutische Zusammensetzung zur oralen Verabreichung mit verlängerter Wirkstofffreisetzung, enthaltend Propiverin und/oder eines oder mehrere seiner pharmazeutisch annehmbaren Salze, wobei die Zusammensetzung die folgende in vitro-Freisetzung, gemessen in 750 ml 0,1N Salzsäure während der ersten Stunde und nachfolgend gemessen in 750 ml USP-Puffer pH = 5,8 unter Anwendung der Ph. Eur. Drehkörbchen-Methode bei 100 U/min und 37 °C, aufweist:

| | | |
|---|---|---|
| 0-20 % | Propiverin, freigesetzt nach | 1 Stunde, |
| 10-45 % | Propiverin, freigesetzt nach | 3 Stunden, |
| 30-60 % | Propiverin, freigesetzt nach | 5 Stunden, |
| 40-75 % | Propiverin, freigesetzt nach | 7 Stunden, |
| 50-80 % | Propiverin, freigesetzt nach | 9 Stunden, |
| 60-90 % | Propiverin, freigesetzt nach | 12 Stunden. |

2. Zusammensetzung nach Anspruch 1, in der eine Menge von 4 mg bis 60 mg Propiverin bzw. die entsprechende Äquivalentmenge eines Propiverinsalzes oder eines Gemisches davon enthalten ist.

3. Zusammensetzung nach einem der Ansprüche 1 oder 2, die zusätzlich mindestens eine oder mehrere saure Substanzen mit einem $pk_a$-Wert von kleiner 6,65, vorzugsweise von 1,8 bis 6,5, enthält.

4. Zusammensetzung nach Anspruch 3, wobei das Äquivalentstoffmengenverhältnis zwischen der Gesamtmenge an einwertig saurer Substanz und Propiverin oder Propiverin-Salz oder Gemischen von diesen 2 : 1 bis 20 : 1, vorzugsweise 3 : 1 bis 10 : 1 beträgt.

5. Zusammensetzung nach einem der Ansprüche 3 oder 4, die als saure Substanz organische Genußsäuren, wie Zitronensäure, Weinsäure. Äpfelsäure, Maleinsäure, Bernsteinsäure, Ascorbinsäure, Fumarsäure, Adipinsäure oder pharmazeutisch annehmbare Salze mehrbasischer Säuren, wie Natrium- oder Kaliumdihydrogencitrat, Dinatrium- oder Dikaliumhydrogencitrat, Natrium- oder Kaliumhydrogentartrat, oder ein Gemisch dieser Säuren und/oder Salze enthält:

6. Zusammensetzung nach einem der Ansprüche 1 bis 5, die mindestens einen retardierenden Stoff, wie Polymere und Copolymere von Acryl- und/oder Methacrylsäurederivaten, wie Eudragit® L, Eudragit® S, Eudragit® RL, Eudragit® RS, Eudragit® E oder Eudragit®NE 30D, Vinylpyrrolidonen, Vinylacetaten, wie Celluloseether, Celluloseester, Polysaccharide, Alginate, Xanthane, Polyvinylalkohole, Celluloseacetatphthalate, Fette, Wachse, Eiweiße oder Schellack enthält.

7. Zusammensetzung nach einem der Ansprüche 1 bis 6 in Form einer Multiple-Unit Formulierung, enthaltend Pellets, Granulat- oder Kompaktteilchen, wobei diese Teilchen aus einem gegebenenfalls retardierten, säurehaltigen Kern bestehen, der mit Propiverin oder Propiverin-Salz und gegebenenfalls mit weiteren Säureanteilen und Hilfsstoffen beschichtet ist und dieser wirkstoffhaltige Kern im weiteren mit einer Retardschicht aus generell magensaft- und darmsaftunlöslichen Polymeren bzw. aus einer Kombination von magen- und darmsaftunlöslichen Polymeren mit magensaftunlöslichen aber darmsaftlöslichen Polymeren umgeben ist und die Zusammensetzung letztendlich in Kapseln oder Sachets abgefüllt oder zusammen mit Tablettierhilfsstoffen zu Tabletten verpresst oder als Bestandteil einer Trinksuspension verwendet wird.

8. Zusammensetzung nach einem der Ansprüche 1 bis 6 in Form einer Multiple-Unit-Formulierung, umfassend Sphäroidtablettenformulierungen, die aus zu Tabletten verpreßten, generell mit magensaftunlöslichem, jedoch darmsaftunlöslichem und/oder darmsaftlöslichem Material überzogenen Granulatteilchen besteht, die wiederum aus einem kompaktierten Gemisch von Propiverin oder Propiverin-Salz, saurer Substanz, Sphäronisierungsmitteln, wie Lactose, mikrokristalliner Cellulose, Hydroxypropylcellulose, Gleitmitteln und Tablettierungsmitteln, wie Polyvinylpyrrolidon, Crosspovidon usw. bestehen, wobei die Tabletten zusätzlich noch mit retardierenden Materialien überzogen sein können.

9. Zusammensetzung nach einem der Ansprüche 1 bis 6 in Form einer Single-Unit-Formulierung, umfassend Matrixtablettenformulierungen, die aus zu Tabletten verpreßten Gemischen von Propiverin oder Propiverin-Salz, gegebenenfalls einer oder mehreren sauren Substanzen, matrixbildenden, retardierenden Hilfsstoffen, wie Celluloseethern oder Celluloseestern, Alginaten, Xanthanen, Fetten und Wachsen oder Polyvinylalkoholen besteht und eventuell mit Überzug aus Polymeren von Acryl- und/oder Methacrylsäurederivaten oder Celluloseethern, Celluloseestern, Vinylacetaten, Vinylpyrrolidonen oder Schellack versehen ist.

## Claims

1. A pharmaceutical composition for oral administration having prolonged release of the active agent, containing propiverine and/or one or several pharmaceutically acceptable salts thereof, having the following in vitro release, measured in 750 ml of 0.1 N hydrochloric acid during the first hour and subsequently measured in 750 ml USP buffer at pH = 5.8 using a Ph. Eur. Basket method at 100 rpm and 37°C:

| | | |
|---|---|---|
| 0 - 20% | propiverine, released after | 1 hour, |
| 10 - 45% | propiverine, released after | 3 hours, |
| 30 - 60% | propiverine, released after | 5 hours, |
| 40 - 75% | propiverine, released after | 7 hours, |
| 50 - 80% | propiverine, released after | 9 hours, |
| 60 - 90% | propiverine, released after | 12 hours. |

2. The composition according to claim 1, wherein an amount of 4 mg to 60 mg of propiverine or the corresponding equivalent amount of a propiverine salt or a mixture thereof is included.

3. The composition according to any one of claims 1 or 2, additionally including at least one or more acidic substances having a $pK_a$ value of less than 6.65, preferably of 1.8 to 6.5.

4. The composition according to claim 3, wherein the molar equvivalent ratio between the total amount of monovalent acidic substance and propiverine or propiverine salt or mixtures thereof is 2:1 to 20:1, preferably 3:1 to 10:1.

**5.** The composition according to any one of claims 3 or 4, including as the acidic substance edible organic acids, such as citric acid, tartaric acid, malic acid, maleic acid, succinic acid, ascorbic acid, fumaric acid, adipic acid or pharmaceutically acceptable salts of multivalent acids, such as sodium or potassium dihydrogencitrate, disodium or dipotassium hydrogencitrate, sodium or potassium hydrogentartrate or a mixture of these acids and/or salts.

**6.** The composition according to any one of claims 1 to 5, including at least one retarding agent, such as polymers and copolymers of acrylic and/or methacrylic acid derivatives, such as Eudragit® L, Eudragit® S, Eudragit® RL, Eudragit® RS, Eudragit® E or Eudragit® NE 30D, vinylpyrrolidones, vinylacetates, such as cellulose ethers, cellulose esters, polysaccharides, alginates, xanthanes, polyvinyl alcohols, cellulose acetate phthalates, fats, waxes, proteins or shellac.

**7.** The composition according to any one of claims 1 to 6, in the form of a multiple-unit formulation containing pellets, granules or compacted particles, wherein these particles are consisting of an optionally retarded, acid containing core which is coated with propiverine or propiverine salt and/or with further acid components and adjuvants and this active agent containing core is surrounded with a retardation layer made of generally gastric fluid and intestinal fluid insoluble polymers or of a combination of gastric fluid and intestinal fluid insoluble polymers with gastric fluid insoluble but intestinal fluid soluble polymers respectively, and wherein the composition is finally filled into capsules or sachets or can be compacted with tabletting adjuvants into tablets or is used as a component of a suspension for drinking.

**8.** The composition according to any one of claims 1 to 6 in the form of a multiple-unit formulation, comprising spheroidal tablet formulations consisting of granular particles coated with gastric fluid insoluble but intestinal fluid insoluble and/or intestinal fluid soluble material in general and being pressed into tablets, wherein the granular particles are consisting of a compacted blend of propiverine or propiverine salt, acidic substance, spheronising agents such as lactose, microcrystaline cellulose, hydroxypropylcellulose, lubricating agents and tabletting adjuvants, such as polyvinylpyrrolidone, crosspovidone etc., and wherein the tablets may be additionally coated with retarding materials.

**9.** The composition according to any one of claims 1 to 6 in the form of a single-unit formulation, comprising matrix tablet formulations consisting of blends of propiverine or propiverine salt, optionally one or several acidic substances, maxtrix-forming retarding adjuvants, such as cellulose ethers or cellulose esters, alginates, xanthanes, fats and waxes or polyvinyl alcohols being pressed into tablets, wherein the formulation is optionally provided with a coating made of polymers of acrylic and/or methacrylic acid derivatives or cellulose ethers, cellulose esters, vinylacetates, vinylpyrrolidones or shellac.

## Revendications

**1.** Composition pharmaceutique destinée à une administration par voie orale à libération prolongée de l'agent actif, contenant de la propivérine et/ou un ou plusieurs de ses sels pharmaceutiquement acceptables, la composition présentant les vitesses de libération suivantes, mesurées dans 750 ml d'acide chlorhydrique 0,1 N pendant la première heure puis dans 750 ml de tampon USP, pH = 5,8 en utilisant la méthode du Panier Rotatif Ph. Eur. À 100 t/min et 37°C :

| | | |
|---|---|---|
| 0 - 20% | propivérine, libérée après | 1 heure |
| 10 - 45% | propivérine, libérée après | 3 heures |
| 30 - 60% | propivérine, libérée après | 5 heures |
| 40 - 75% | propivérine, libérée après | 7 heures |
| 50 - 80% | propivérine, libérée après | 9 heures |
| 60 - 90% | propivérine, libérée après | 12 heures. |

**2.** Composition selon la revendication 1, dans laquelle est contenue une quantité de 4 mg à 60 mg de propivérine ou bien la quantité équivalente d'un sel de propivérine ou d'un mélange de ceux-ci.

**3.** Composition selon l'une des revendications 1 ou 2, qui contient en outre au moins une ou plusieurs substances acides avec une valeur $pK_a$ inférieure à 6,65, de préférence de 1,8 à 6,5.

**4.** Composition selon la revendication 3, le rapport de quantités équivalentes entre la quantité totale de substance acide monovalente et de propivérine, d'un sel de propivérine ou de mélanges de ceux-ci étant de 2:1 à 20:1, de préférence de 3:1 à 10:1.

**5.** Composition selon l'une des revendications 3 ou 4, qui contient, en tant que substances acides, des acides alimentaires comme l'acide citrique, l'acide tartrique, l'acide malique, l'acide maléique, l'acide succinique, l'acide ascorbique, l'acide fumarique, l'acide adipique ou des sels pharmaceutiquement acceptables, des acides polybasiques tels que le citrate de dihydrogène de sodium ou de potassium ou bien un mélange de ces acides et/ou de ces sels.

**6.** Composition selon l'une des revendications 1 à 5, qui contient au moins un agent retardant comme des polymères ou copolymères de dérivés de l'acide acrylique et/ou méthacrylique, comme l'Eudragit® L, l'Eudragit® S, l'Eudragit® RL, l'Eudragit® RS, l'Eudragit® E ou l'Eudragit® NE 30D, des vinylpyrrolidones, des acétates de vinyles comme des éthers de cellulose, des esters de cellulose, des polysaccharides, des alginates, des xanthanes, des alcools polyvinyliques, des phthalates d'acétates de cellulose, des graisses, des cires, des protéines ou de la gomme laque.

**7.** Composition selon l'une des revendications 1 à 6 sous la forme d'une formulation à unités multiples, contenant des pastilles, des particules granulées ou compactes, ces particules étant constituées d'un noyau contenant des acides, le cas échéant retardé, enrobé de propivérine ou d'un sel de propivérine et le cas échéant d'autres substances acides et agents auxiliaires et ce noyau contenant l'agent actif est en outre entouré d'une couche de retardement généralement constituée de polymères insolubles dans les sucs gastriques et intestinaux ou d'une combinaison de polymères insolubles dans les sucs gastriques et intestinaux et de polymères insolubles dans les sucs gastriques mais solubles dans les sucs intestinaux et la composition est ensuite introduite dans des capsules ou des sachets ou bien réalisée sous la forme de pastilles avec des agents auxiliaires pour la fabrication de pastilles ou utilisée en tant que composant d'une suspension buvable.

**8.** Composition selon l'une des revendications 1 à 6 sous la forme d'une formulation à unités multiples, comprenant des formulations à pastilles sphéroïdes, constituée de particules granulées généralement enrobées d'un matériau insoluble dans les sucs gastriques et insolubles dans les sucs intestinaux et/ou solubles dans les sucs intestinaux, comprimées sous forme de pastilles, ces particules étant constituées d'un mélange compacté de propivérine ou d'un sel de propivérine, d'une substance acide, d'agent de sphéronisation comme du lactose, de la cellulose microcristalline, de l'hydroxypropylcellulose, des agents lubrifiants et des agents de formation de pastilles comme de la polyvinylpyrrolidone, de la crosspovidone etc., les pastilles pouvant en outre être enrobées de matériaux retardants.

**9.** Composition selon l'une des revendications 1 à 6 sous la forme d'une formulation à unité unique, comprenant des formulations à pastilles matricielles constituées de mélanges de propivérine ou d'un sel de propivérine, le cas échéant d'une ou plusieurs substances acides, d'agents auxiliaires matriciels et retardants, comme des éthers de cellulose ou des esters de cellulose, des alginates, des xanthanes, des graisses et des cires ou des alcools polyvinyliques, éventuellement munie d'un enrobage de polymères de dérivés d'acide acrylique et/ou méthacrylique ou d'éthers de cellulose, d'esters de cellulose, d'acétates de vinyle, de vinylpyrrolidones ou de gomme laque.

Abbildung 1

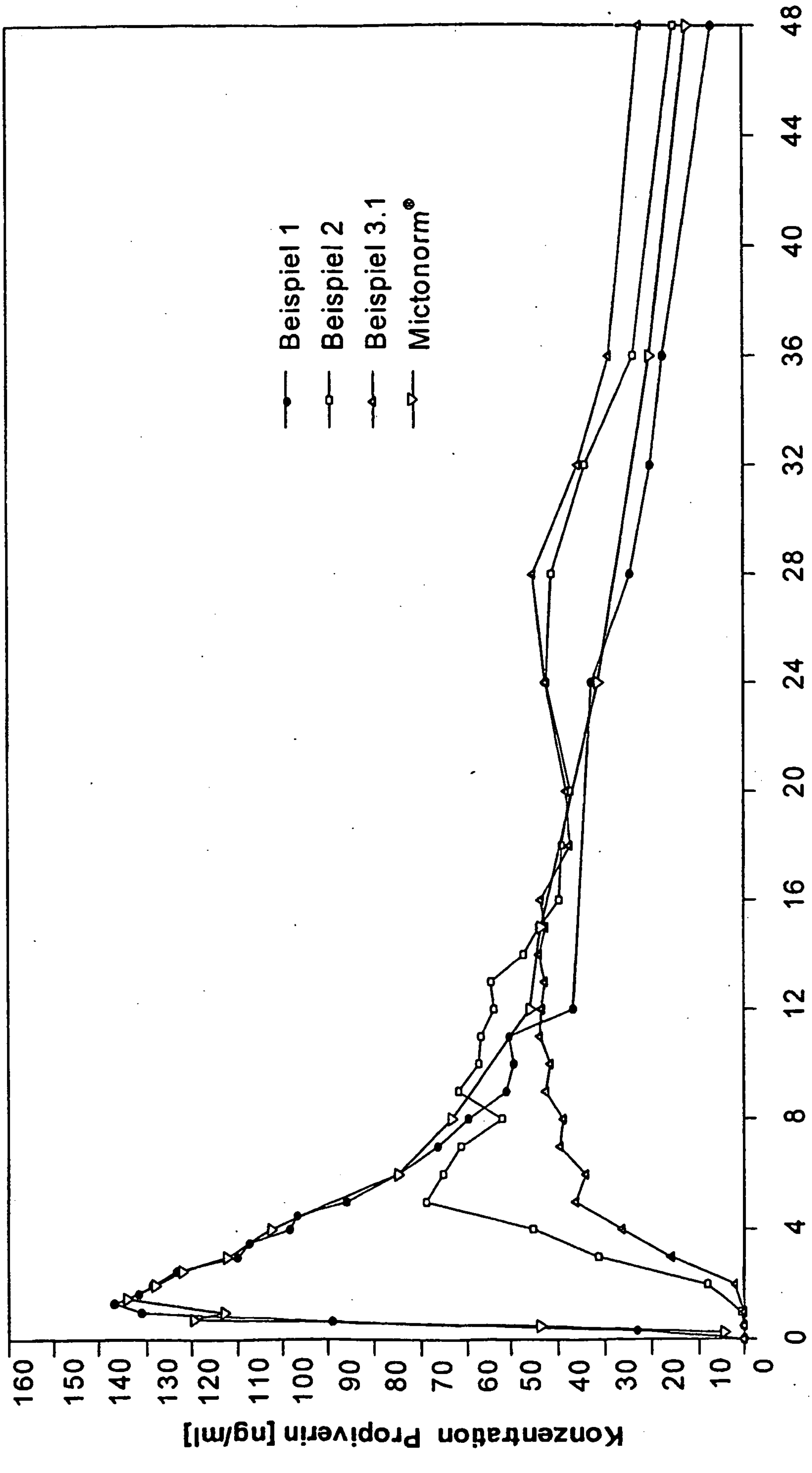
Beispiel 1
Beispiel 2
Beispiel 3.1
Mictonorm®
Konzentration Propiverin [ng/ml]
160
150
140
130
120
110
100
90
80
70
60
50
40
30
20
10
0
0
4
8
12
16
20
24
28
32
36
40
44
48

Abbildung 2

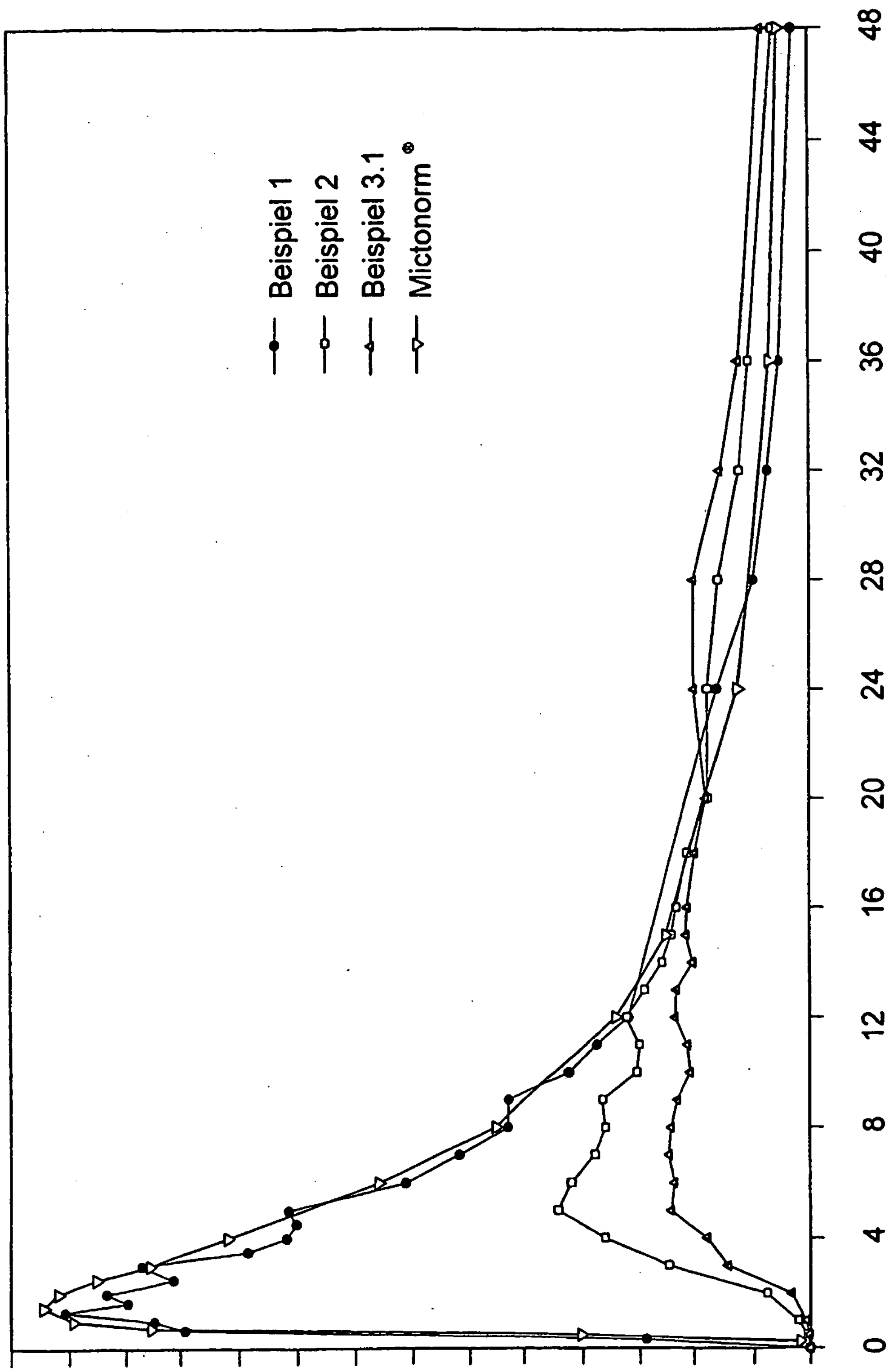

Beispiel 1
Beispiel 2
Beispiel 3.1
Mictonorm ®
Konzentration Propiverin-N-Oxid [ng/ml]
1400
1300
1200
1100
1000
900
800
700
600
500
400
300
200
100
0
Zeit [h]
0
4
8
12
16
20
24
28
32
36
40
44
48

Abbildung 3

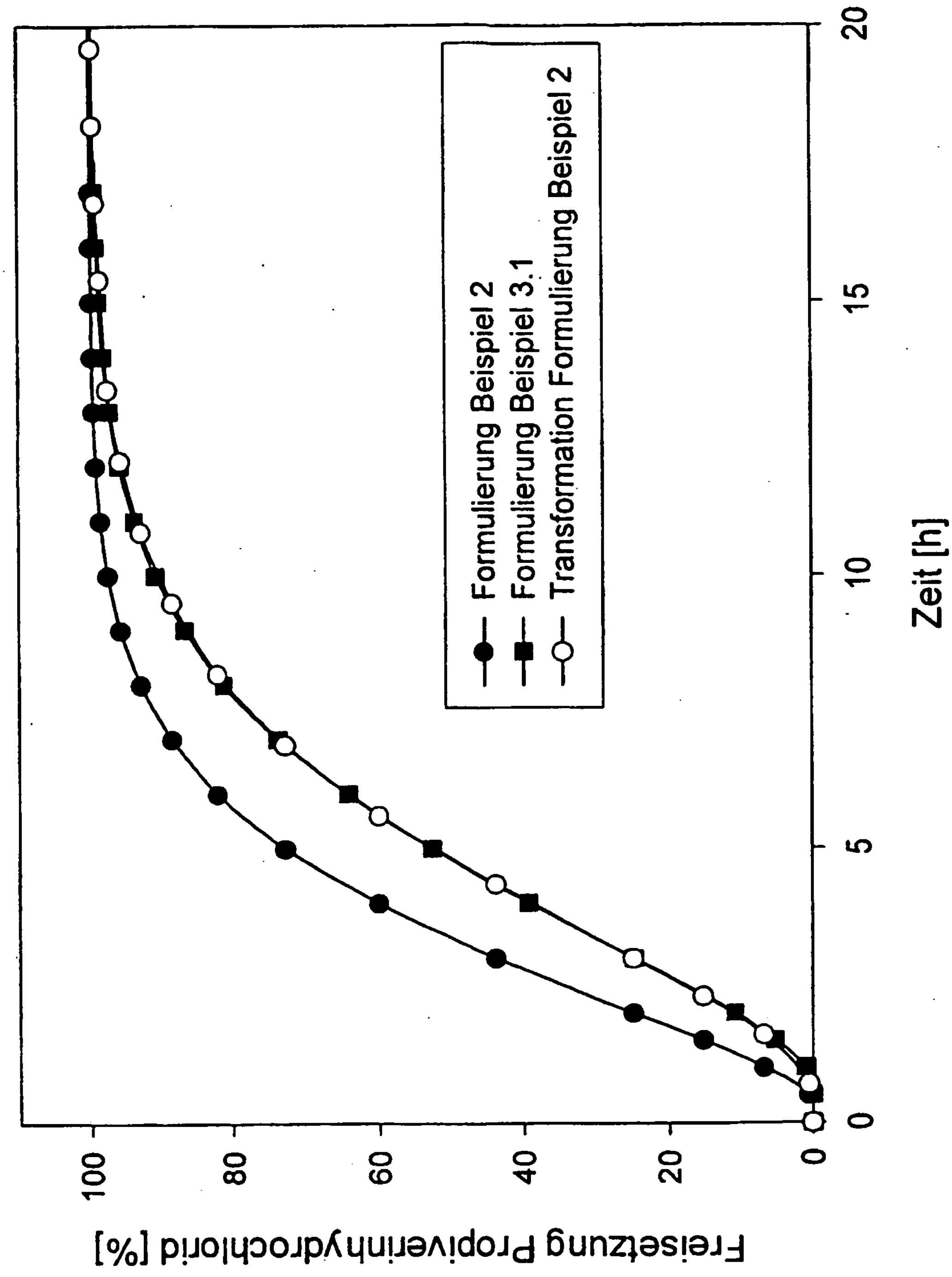

Formulierung Beispiel 2
Formulierung Beispiel 3.1
Transformation Formulierung Beispiel 2
Zeit [h]
Freisetzung Propiverinhydrochlorid [%]
0
5
10
15
20
0
20
40
60
80
100

Abbildung 4

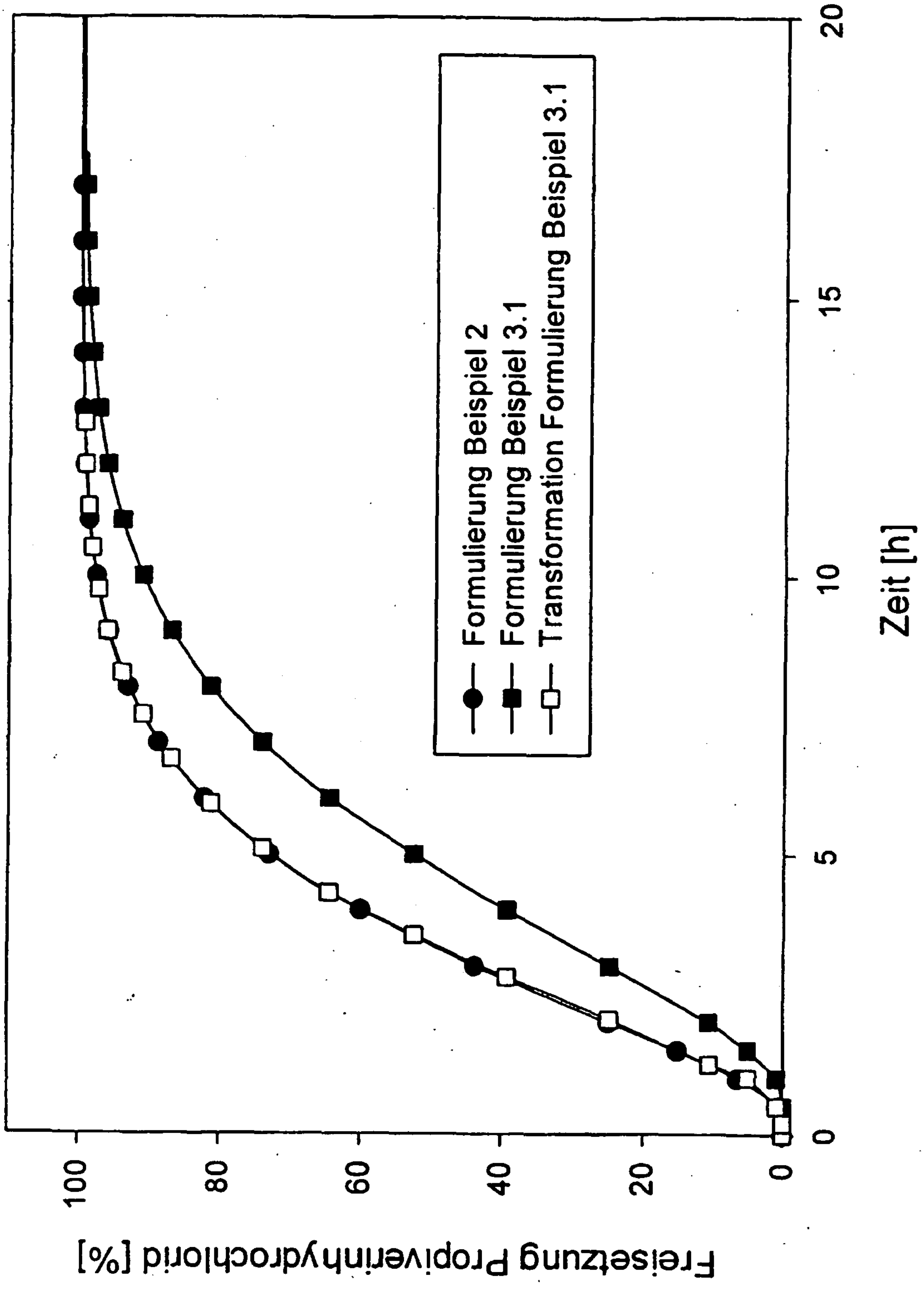
Formulierung Beispiel 2
Formulierung Beispiel 3.1
Transformation Formulierung Beispiel 3.1
Freisetzung Propiverinhydrochlorid [%]
0
20
40
60
80
100
Zeit [h]
0
5
10
15
20

Abbildung 5

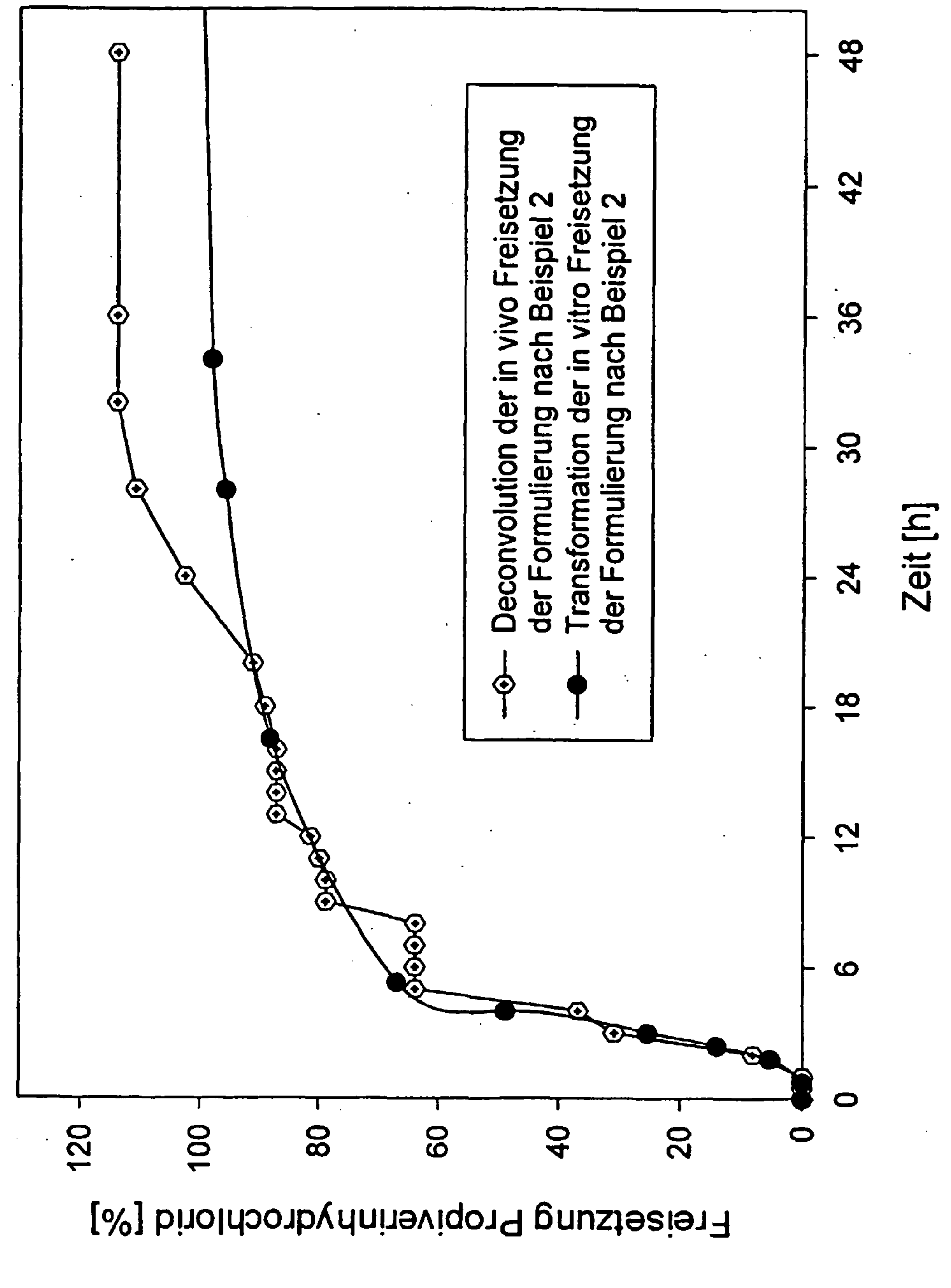
Deconvolution der in vivo Freisetzung der Formulierung nach Beispiel 2
Transformation der in vitro Freisetzung der Formulierung nach Beispiel 2
Freisetzung Propiverinhydrochlorid [%]
0
20
40
60
80
100
120
Zeit [h]
0
6
12
18
24
30
36
42
48

Abbildung 6

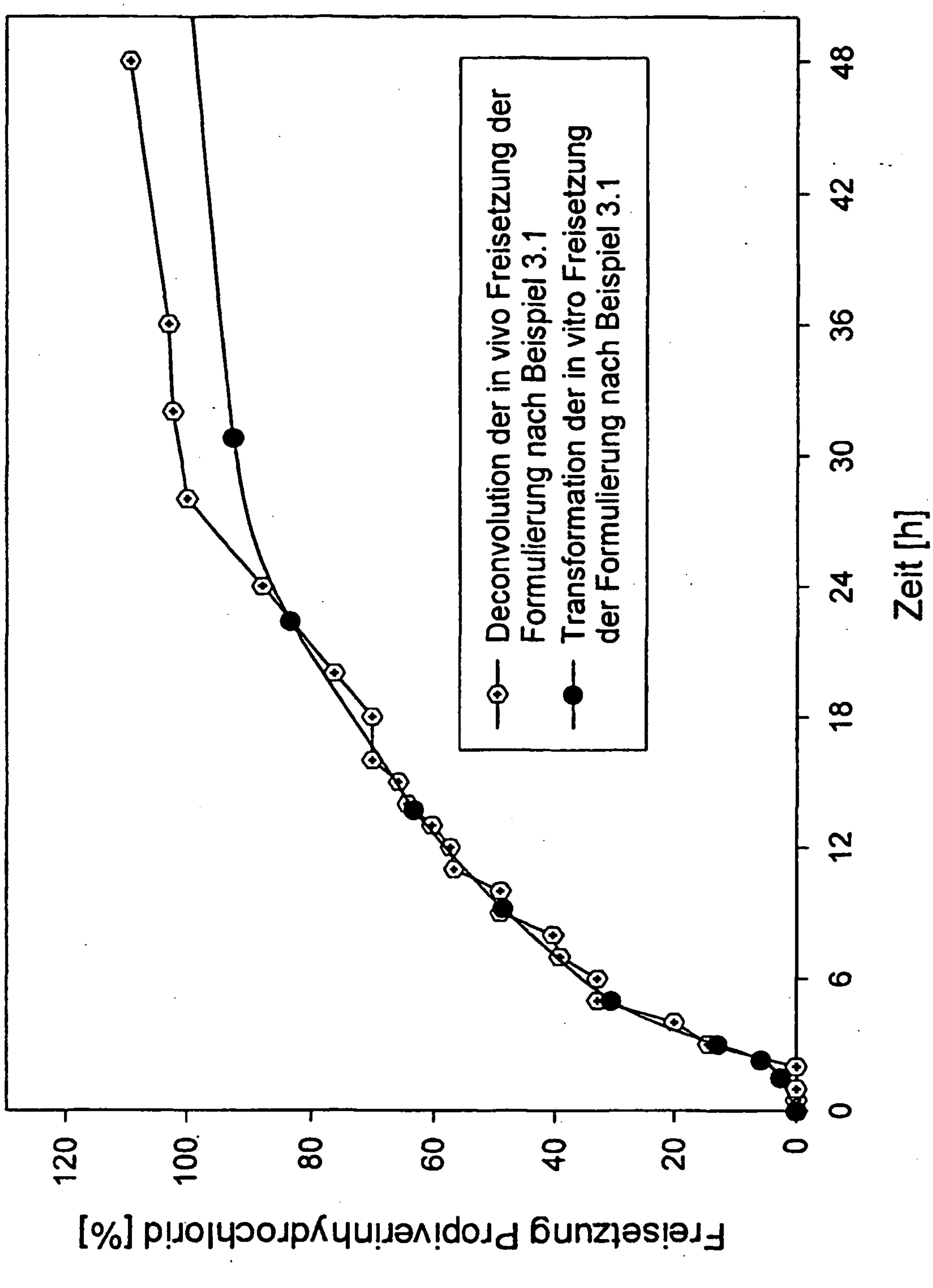
Deconvolution der in vivo Freisetzung der Formulierung nach Beispiel 3.1
Transformation der in vitro Freisetzung der Formulierung nach Beispiel 3.1
Freisetzung Propiverinhydrochlorid [%]
0
20
40
60
80
100
120
Zeit [h]
0
6
12
18
24
30
36
42
48

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2937589
- US 5912268 A
- WO 9523593 A
- WO 9612477 A
- WO 9637202 A
- WO 0012069 A
- WO 0027369 A
- EP 32562 A
- EP 68191 A
- EP 69259 A
- WO 0044353 A


### In der Beschreibung aufgeführte Nicht-Patentliteratur


- *Biol. Pharm. Bull.,* 1995, vol. 18 (7), 968-975
- *Int. J. Pharm.,* 1997, vol. 157, 181-187
- *Pharm. Ind.,* 1991, vol. 53, 686-690
- *Pharm. Ind.,* 1989, vol. 51, 98-101540-543
- *ebenda,* 1991, vol. 53, 69-73595-600778-785
- *Arzneim.-Forsch./Drug Res.,* 1998, vol. 48, 540-604